# EUROPEAN PATENT APPLICATION

(11) **EP 4 644 541 A1**
(43) Date of publication of application: **05.11.2025**
(21) Application number: 23913004.0
(22) Date of filing: 28.12.2023
(51) Int. Cl.: C12N 9/78, C12N 9/22, C12N 15/62, C12N 15/86, A61K 48/00, A61K 31/7088, A61P 27/02

(54) **MITOCHONDRIAL BASE MUTATION EDITING SYSTEM FOR LEBER HEREDITARY OPTIC NEUROPATHY**

(30) Priority: 29.12.2022 KR 20220189900
(71) Applicant: Edgene, Inc., Incheon 21990 (KR)
(72) Inventor: KIM, Jin Soo, Seoul 08505 (KR); LEE, Seong Hyun, Seoul-si 08505 (KR); LIM, Chae Jin, Seoul 08505 (KR)
(74) Representative: Berggren Oy
(86) International application number: PCT/KR2023/021945
(87) International publication number: WO 2024/144330

(57) **Abstract**

The present invention relates to a base editing system for correcting mutations G3460A, G11778A, or T14484C in mitochondrial DNA of a patient with Leber hereditary optic neuropathy (LHON) to a normal genotype. Specifically, the present invention provides a method for correcting a mutation in the mitochondrial genes of a patient with LHON to a normal genotype using a base editor that recognizes specific sites in the mitochondrial genes of the patient with LHON and has an activity of specifically correcting the adenine base at position 3460 or 11778, or the cytosine base at position 14484, by using a fusion protein or a polynucleotide encoding such a fusion protein. The base editor or nucleotide according to the invention may correct DNA mutations specific to LHON in a cellular or extracellular in vitro environment and, more preferably, may be used as a gene therapy agent for the prevention or treatment of the disease. Thus, the present invention also provides a use of the substance in the prevention or treatment of LHON.

## Description

### [Technical Field]

The present invention relates to a base editing system for correcting mutations G3460A, G11778A, or T14484C in mitochondrial DNA of a patient with Leber hereditary optic neuropathy (LHON) to a normal genotype. Specifically, the present invention provides a method for correcting a mutation in the mitochondrial genes of a patient with LHON to a normal genotype using a base editor that recognizes specific sites in the mitochondrial genes of the patient with LHON and has an activity of specifically correcting the adenine base at position 3460 or 11778, or the cytosine base at position 14484, by using a fusion protein or a polynucleotide encoding such a fusion protein. The base editor or polynucleotide according to the present invention can exert an effect of correcting DNA mutations specific to LHON in a cellular or extracellular in vitro environment and, more preferably, can be used as a gene therapy agent capable of preventing or treating the disease. Thus, the present invention also provides a use of the substance in the prevention or treatment of LHON.

### [Background Art]

Mitochondria are organelles within eukaryotic cells that produce energy (ATP) used in biological processes, and have genes that are independent of the nuclear genes. More than 80% of ATP, the energy source used by cells, is produced by mitochondria, and mutations in the mitochondrial genome (mitochondrial DNA, mtDNA) may cause fatal defects in the central nervous system, heart, muscles, visual and auditory functions, and the like. mtDNA is inherited maternally, so if there are mutations in the mother's mitochondrial DNA, they are passed on to the next generation. Most patients diagnosed with mtDNA mutations inherit the mutations from the maternal line, and approximately 40% of these are reported to arise spontaneously. Mutations in mtDNA, which cause mitochondrial diseases, occur in approximately 1 in 5,000 people. Genetic diseases caused by mutations in mitochondrial DNA are very diverse, but most of them lack effective treatments or preventive measures. Representative mitochondrial genetic disorders include Leber hereditary optic neuropathy (LHON), mitochondrial encephalopathy, lactic acidosis, and stroke-like episode (MELAS), Leigh syndrome, and the like.

Among the various mitochondrial disorders, LHON is the first genetic disease identified as being caused by mutations in mitochondrial genes, and was first described in 1871 by German ophthalmologist Theodore Leber. LHON is also called Leber's optic atrophy. Unlike other diseases, LHON is characterized by its onset and rapid progression without any distinct prodromal symptoms or pain, and may occur at any age. It is known to mainly occur in men in their 20s and 30s, with the average age of onset being 20 to 30, and most patients experience complete loss of vision in both eyes simultaneously or consecutively after a few months.

LHON is a major disease, accounting for approximately 30 to 50% of idiopathic optic neuropathies that cause vision loss in both eyes. Patients with LHON have a G→A substitution at base 3460 of the ND1 gene in mtDNA, a G→A substitution at base 11778 of the ND4 gene, or a T→C substitution at base 14484 of the ND6 gene, which causes functional impairment of complex 1, which is composed of proteins encoded by the corresponding genes, and these three point mutations account for more than 90% of LHON onset. In conclusion, LHON is known as a major cause of bilateral unexplained optic neuropathy.

### [Disclosure]

### [Technical Problem]

Despite extensive research on the LHON genetic disorder, there is no appropriate treatment, and patients remain untreated without any effective alternative treatment and eventually lose their vision. At the time of the present invention, the only treatment for LHON was idebenone, which was developed by Santhera Pharmaceuticals and approved under the trade name Raxone, and although it delays the progression of blindness, it has a limitation in that it is not a fundamental cure. Accordingly, the purpose of the present invention is to provide a base editor for correcting mutations in mitochondrial DNA that causes LHON, and thereby provide a method for preventing or treating LHON using the same.

### [Technical Solution]

A base editing system according to the present invention uses an adenine base editor capable of correcting A at position 3460 of mitochondrial DNA of a patient with LHON to G, an adenine base editor capable of correcting A at position 11778 to G, or a cytosine base editor capable of correcting C at position 14484 to T. The above base editor utilizes a combination of one or more fusion proteins or polynucleotides encoding the one or more fusion protein, wherein the one or more fusion proteins each independently comprise a DNA binding protein and further comprise a deaminase (at least one of adenine deaminase and cytosine deaminase). The DNA binding protein used in the base editing system according to the present invention may be a zinc finger protein (also called "ZF"), a transcriptional activator-like effector (TALE) protein, or a CRISPR-associated nuclease, or a combination thereof, and the deaminase may be an apolipoprotein B editing complex (APOBEC), an activation induced deaminase (AID), a tRNA-specific adenosine deaminase (TadA) or a variant thereof, and DddAₜₒₓ or a variant thereof (existing in the form of full-length or two split units), or a combination thereof. The fusion protein used in the base editing system according to the present invention may additionally include one or more of UGI (uracil glycosylase inhibitor), NES (nuclear export signal), and MTS (mitochondrial targeting sequence). The base editor according to the present invention may have the form of a composition of one or more fusion proteins as described above or a polynucleotide encoding the one or more fusion proteins. Prior to the present invention, there was no known method for preventing or treating LHON by correcting, through base editing, point mutations in mitochondrial genes that occur in a patient with LHON to a normal genotype.

### [Description of Drawings]

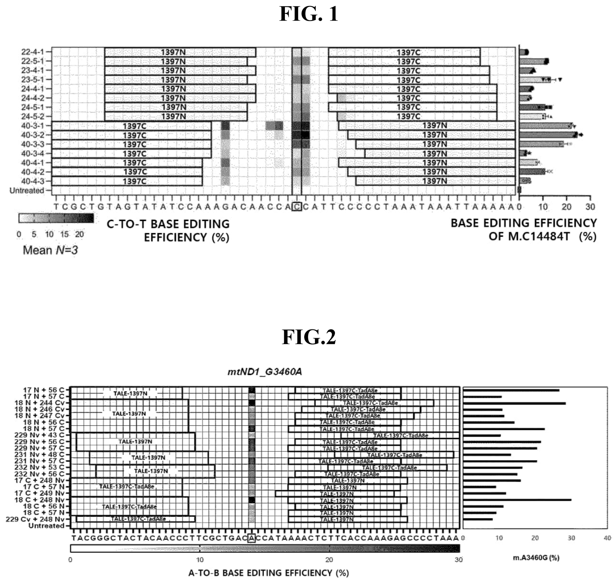

FIG. 1 shows the results of screening the editing efficiency of m.C14484T using DdCBE (DddA-derived cytosine base editor). 1397N represents the N-terminal split of G1397 DddAₜₒₓ, and 1397C represents the C-terminal split of G1397 DddAₜₒₓ. A boxed portions on the left side represents a DNA sequence recognized by a first fusion protein comprising 1397N or 1397C, and a boxed portion on the right side represents a DNA sequence recognized by a second fusion protein comprising 1397N or 1397C. The boxed "C" indicates the base at position 14484 (T14484C) to be corrected, and a degree of boldness represents the efficiency of correction from C to T. The bar graphs on the right show the frequency of 14484C→T corrections.
FIG. 2 shows the results of screening the editing efficiency of m.A3460G using TALED (TALE-linked deaminase). 1397N represents the N-terminal split of G1397 DddAₜₒₓ, and 1397C represents the C-terminal split of G1397 DddAₜₒₓ. A boxed portion on the left side represents a DNA sequence recognized by a first fusion protein comprising TALE and 1397N, or TALE, 1397C, and TadA8e, and a boxed portion on the right side represents a DNA sequence recognized by a second fusion protein comprising TALE and 1397N, or TALE, 1397C, and TadA8e. The boxed "A" indicates the base at position 3460 (G3460A) to be corrected, and the degree of boldness represents the efficiency of correction from A to G. The bar graphs on the right show the frequency of 3460A→G corrections.

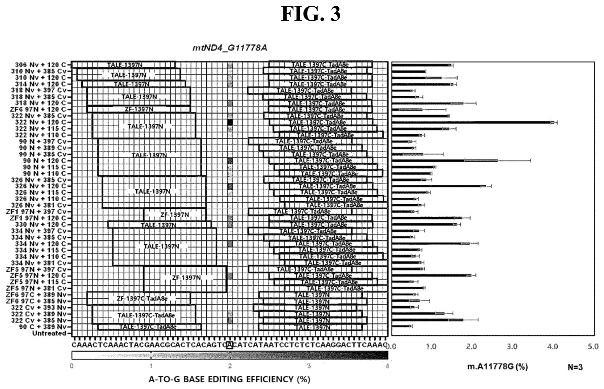

FIG. 3 shows the results of screening the efficiency of m.A11778G correction using TALED and ZFD (zinc finger deaminase). 1397N represents the N-terminal split of G1397 DddAₜₒₓ, 1397C represents the C-terminal split of G1397 DddAₜₒₓ, and ZF represents a zinc finger protein. A boxed portion on the left side represents a DNA sequence recognized by a first fusion protein comprising TALE or ZF protein and 1397N, or TALE or ZF protein, 1397C and TadA8e, and the boxed portion on the right side represents a DNA sequence recognized by a second fusion protein comprising TALE and 1397N, or TALE, 1397C and TadA8e. The boxed "A" indicates the base at position 11778 (G11778A) to be corrected, and the degree of boldness represents the efficiency of correction from A to G. The bar graphs on the right show the frequency of 11778A→G corrections.

### [Best Mode for Carrying Out the Invention]

Unless defined otherwise, all technical and scientific terms used herein have the same meaning as commonly understood by one of ordinary skill in the art to which this invention belongs. In general, the terms used herein are well known and commonly used in the art.

The terms "correct," "edit," and "editing" as used herein are used interchangeably and refer to a method of altering a nucleic acid sequence by selective mutation of a specific genomic target. Such a specific genomic target includes, but are not limited to, a gene, a promoter, an open reading frame or any nucleic acid sequence.

The term "base editor" or "mitochondrial DNA base editor" as used herein means a substance capable of altering a nucleic acid sequence by selective mutation of a mitochondrial genome target, and includes a combination of one or more different base editors. The term "base editor" or "mitochondrial DNA base editor" as used herein may be in the form of a polypeptide (which may be a fusion protein) or a polynucleotide, depending on the context, and may be a composition comprising one or more polypeptides (which may be fusion proteins) or polynucleotides. That is, the term "base editing composition" as used herein means a combination of one or more different base editors, wherein the different base editors may be used simultaneously or separately.

The term "target" or "target site" as used herein means a pre-identified nucleic acid sequence of any composition and/or length. Such a target site includes, but are not limited to, a gene, a promoter, an open reading frame or any nucleic acid sequence.

The present invention relates to a base editor capable of editing a mitochondrial DNA mutation of a patient with Leber hereditary optic neuropathy (LHON), wherein the base editor comprises one or more fusion proteins, wherein the one or more fusion proteins each independently comprise a DNA binding protein that specifically binds to the mitochondrial DNA of the patient with LHON, and may have a form of a composition additionally comprising one or more of adenine deaminase and cytosine deaminase. The cytosine deaminase may be present in a full-length form or in the form of two splits.

The base editor according to the present invention is capable of editing adenine (A) at position 3460 of mitochondrial ND1 DNA of a patient with LHON to guanine (G), adenine (A) at position 11778 of mitochondrial ND4 DNA to guanine (G), or cytosine (C) at position 14484 of mitochondrial ND6 DNA to thymine (T).

In this specification, it is apparent to a person skilled in the art that the base at position 3460 of ND1 DNA is referred to the 3460th base among all bases constituting the mitochondrial DNA and indicates a base constituting the ND1 DNA, and the base at position 11778 of ND4 DNA is referred to the 11778th base among all bases constituting the mitochondrial DNA and indicates a base constituting the ND4 DNA, and the base at position 14484 of ND6 DNA is referred to the 14484th base among all bases constituting the mitochondrial DNA and indicates a base constituting the ND6 DNA.

The cytosine deaminase that may be used in the base editor according to the present invention means an amino group deaminase capable of converting a cytosine into uridine, and may be derived from or mutated (e.g., engineered or evolved) from any organism (e.g., eukaryotes or prokaryotes) including, but not limited to, algae, bacteria, fungi, plants, invertebrates, and mammals. For example, it may be a cytosine deaminase derived from or mutated from APOBEC (apolipoprotein B editing complex), AID (activation-induced deaminase), TadA (tRNA-specific adenosine deaminase), a bacterial adenine deaminase, or an ortholog thereof, or a cytosine deaminase derived from or mutated by DddA, a bacterial cytosine deaminase, or an ortholog thereof, or a fragment thereof. The cytosine deaminase mutated from the above-mentioned TadA may be, for example, one in which one or more of the amino acid residues 6, 26, 27, 28, 46, 48, 49, 61, 74, 76, 77, 82, 96, 107, 108, 112, 114, 115, 119, 122, 127, 142, 143, 151, 154 and 158 of the amino acid sequence of SEQ ID NO: 1 are mutated to another amino acid. For example, it may be a polypeptide in which the 27th amino acid in the amino acid sequence of SEQ ID NO: 1 is mutated to lysine, the 28th amino acid is mutated to alanine, the 61st amino acid is mutated to isoleucine, and the 96th amino acid is mutated to asparagine. Regarding the composition of the cytosine deaminase that may be used in the present invention, reference may be made to international patent application publications WO 2022/060185, WO 2023/086953, and the like, which are incorporated by reference in their entirety into this application, and which were already known prior to the present application.

When the base editor according to the present invention includes cytosine deaminase, the cytosine deaminase may be included in the form of a first split and a second split, and may also be included in a full-length form. In the case where the first split and the second split are provided, the first split and the second split may each be in a form linked to a DNA binding protein.

In this specification, when two proteins are said to be "linked," the two proteins may be directly linked or indirectly linked via a linker or other protein(s).

The cytosine deaminase as used in the present invention may be DddAₜₒₓ, which is a portion of a bacterial toxin derived from Burkholderia cenocepacia that exhibits an enzymatic function and may deaminate cytosine of double-stranded DNA. DddAₜₒₓ may comprise the amino acid sequence of SEQ ID NO: 2.

SEQ ID NO: 2: wild-type DddAₜₒₓ

GSYALGPYQISAPQLPAYNGQTVGTFYYVNDAGGLESKVFSSGGPTPYPNYA

NAGHVEGQSALFMRDNGISEGLVFHNNPEGTCGFCVNMTETLLPENAKMTVVPPEG

AIPVKRGATGETKVFTGNSNSPKSPTKGGC

Since DddAₜₒₓ is toxic to cells, it may be used in the form of two inactive splits, namely a first split and a second split, to avoid toxicity in a host cell. When the cytosine deaminase as used in the present invention is used in the form of a first split and a second split, each of the first split and the second split has no deamination activity.

The first split of the DddAₜₒₓ cytosine deaminase may comprise a sequence from the N-terminus to G33, G44, A54, N68, G82, N98, or G108 of the amino acid sequence of SEQ ID NO: 2, and the second split may comprise a sequence from G34, P45, G55, N69, T83, A99, or A109 of the amino acid sequence of SEQ ID NO: 2 to the C-terminus.

Preferably, the first split of the DddAₜₒₓ cytosine deaminase may comprise a sequence from the N-terminus to G44 of the amino acid sequence of SEQ ID NO: 2 (SEQ ID NO: 3 below), and the second split may comprise a sequence from P45 to the C-terminus (SEQ ID NO: 4 below).
SEQ ID NO: 3: wild-type DddAₜₒₓ G1333-N
   GSYALGPYQISAPQLPAYNGQTVGTFYYVNDAGGLESKVFSSGG
SEQ ID NO: 4: wild-type DddAₜₒₓ G1333-C

PTPYPNYANAGHVEGQSALFMRDNGISEGLVFHNNPEGTCGFCVNMTETLLP

ENAKMTVVPPEGAIPVKRGATGETKVFTGNSNSPKSPTKGGC

Preferably, the first split of the DddAₜₒₓ cytosine deaminase may comprise the sequence from the N-terminus to G108 of the amino acid sequence of SEQ ID NO: 2 (SEQ ID NO: 5 below), and the second split may comprise the sequence from A109 to the C-terminus (SEQ ID NO: 6 below).
SEQ ID NO: 5: wild-type DddAₜₒₓ G1397-N 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
SEQ ID NO: 6: wild-type DddAₜₒₓ G1397-C
   AIPVKRGATGETKVFTGNSNSPKSPTKGGC

When the first split and the second split of DddAₜₒₓ are used as cytosine deaminase, one or more amino acids located at the surface where the first split and the second split of the cytosine deaminase bind to each other may be substituted with other amino acids. For example, the first split and the second split of DddAₜₒₓ may each comprise the amino acid sequences of SEQ ID NO: 3 (G1333-N) and SEQ ID NO: 4 (G1333-C), in which case, at least one amino acid selected from the group consisting of positions 3, 5, 10, 11, 13, 14, 15, 16, 17, 18, 19, 28, 30 and 31 of SEQ ID NO: 3 or at least one amino acid selected from the group consisting of positions 13, 16, 17, 20, 21, 28, 29, 30, 31, 32, 33, 56, 57, 58 and 60 of SEQ ID NO: 4 may be substituted with another amino acid, but is not limited thereto. In another example, the first split of DddAₜₒₓ may comprise the amino acid sequence of SEQ ID NO: 5 (G1397-N) and SEQ ID NO: 6 (G1397-C), wherein at least one amino acid selected from the group consisting of positions 87, 88, 91, 92, 95, 100, 101, 102 and 103 of SEQ ID NO: 5 or at least one amino acid selected from the group consisting of positions 13, 14, 15 and 16 of SEQ ID NO: 6 may be substituted with another amino acid, but is not limited thereto. The "another amino acid" refers to an amino acid selected from among alanine, isoleucine, leucine, methionine, phenylalanine, proline, tryptophan, valine, aspartic acid, cysteine, glutamine, glycine, serine, threonine, tyrosine, aspartic acid, glutamic acid, arginine, histidine, lysine, and all known variants of the above amino acids, excluding the amino acid that the wild-type protein originally has at the mutation position. Using such a variant, when the pair of DddAₜₒₓ splits, each linked to a DNA-binding protein, fails to bind to DNA, the pair does not function properly, thereby causing highly efficient and precise C-to-T correction without undesired off-target C-to-T correction. As examples of the variant, there may be provided a first split of DddAₜₒₓ having the amino acid sequence of SEQ ID NO: 139 (which may be referred to as "G1397-N" or "G1397N") and a second split of DddAₜₒₓ having the amino acid sequence of SEQ ID NO: 140 (which may be referred to as "G1397-C" or "G1397C").

In the present specification, the term "G1333-N", "G1333N" or "1333N" may refer to a first split of wild-type DddAₜₒₓ having an amino acid sequence of SEQ ID NO: 3 or an amino acid variant thereof, and the term "G1333-C", "G1333C" or "1333C" may refer to a second split of wild-type DddAₜₒₓ having an amino acid sequence of SEQ ID NO: 4 or an amino acid variant thereof.

In the present specification, the term "G1397-N", "G1397N" or "1397N" may refer to a first split of wild-type DddAₜₒₓ having an amino acid sequence of SEQ ID NO: 5 or 139 or an amino acid variant thereof, and the term "G1397-C", "G1397C" or "1397C" may refer to a second split of wild-type DddAₜₒₓ having an amino acid sequence of SEQ ID NO: 6 or 140 or an amino acid variant thereof.

The cytosine deaminase as used in the present invention may be used in a full-length form, and the full-length cytosine deaminase (e.g., DddAₜₒₓ) used in this case has an amino acid sequence that is modified to reduce or eliminate toxicity. The C-terminus of DddAₜₒₓ is specifically enriched with positively charged amino acids. Because DNA is negatively charged, it binds to positively charged amino acids in proteins. By substituting this positively charged amino acid, the binding strength of DddAₜₒₓ to DNA may be weakened, thereby reducing or eliminating intracellular toxicity. That is, if a positively charged amino acid is substituted to eliminate the toxicity, cloning using E. coli is possible, thereby securing full-length DddAₜₒₓ. Such non-toxic full-length cytosine deaminase may be provided by substituting one or more, two or more, three or more, four or more, or five or more amino acids of the wild-type amino acid sequence of SEQ ID NO: 2 with another amino acid. The "another amino acid" refers to an amino acid selected from among alanine, isoleucine, leucine, methionine, phenylalanine, proline, tryptophan, valine, aspartic acid, cysteine, glutamine, glycine, serine, threonine, tyrosine, aspartic acid, glutamic acid, arginine, histidine, lysine, and all known variants of the above amino acids, excluding the amino acid that the wild-type protein originally has at the mutation position. For example, the another amino acid may be alanine.

The non-toxic full-length DddAₜₒₓ may comprise an amino acid sequence selected from the group consisting of the following amino acid sequences.
A1341D KRKKA Variant 
GSYALGPYQISAPQLPAYNGQTVGTFYYVNDAGGLESKVFSSGGPTPYPNYD
NAGHVEGQSALFMRDNGISEGLVFHNNPEGTCGFCVNMTETLLPENAKMTVVPPEG
AIPVKRGATGETKVFTGNSNSPKSPTAGGC
AAAAA Variant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVAAGATGETAVFTGNSNSPASPTAGGC
AAAAK Variant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVAAGATGETAVFTGNSNSPASPTKGGC
AAKAA Variant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVAAGATGETKVFTGNSNSPASPTAGGC
AAKAK Variant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVAAGATGETKVFTGNSNSPASPTKGGC
KAAAA Variant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVKAGATGETAVFTGNSNSPASPTAGGC
E1347A Variant

GSYALGPYQISAPQLPAYNGQTVGTFYYVNDAGGLESKVFSSGGPTPYPNYA
NAGHVAGQSALFMRDNGISEGLVFHNNPEGTCGFCVNMTETLLPENAKMTVVPPEG
AIPVKRGATGETKVFTGNSNSPKSPTKGGC

Preferably, the full-length cytosine deaminase variant that may be used in the present invention may have one or more amino acid substitutions selected from the group consisting of a substitution of S at position 37 to G, a substitution of G at position 59 to S, a substitution of A at position 109 to V, and a substitution of S at position 129 to G in the amino acid sequence of SEQ ID NO: 2.

More preferably, the full-length cytosine deaminase variant that may be used in the present invention may have all of the substitution of S at position 37 to G, the substitution of G at position 59 to S, the substitution of A at position 109 to V and the substitution of S at position 129 to G in the amino acid sequence of SEQ ID NO: 2, in which case the sequence is as follows.
GSVG Variant

GSYALGPYQISAPQLPAYNGQTVGTFYYVNDAGGLEGKVFSSGGPTPYPNYA
NAGHVESQSALFMRDNGISEGLVFHNNPEGTCGFCVNMTETLLPENAKMTVVPPEG
VIPVKRGATGETKVFTGNSNGPKSPTKGGC

In another example, the full-length cytosine deaminase variant that may be used in the present invention may comprise the following sequences.
SSVG Variant 
GSYALGPYQISAPQLPAYNGQTVGTFYYVNDAGGLESKVFSSGGPTPYPNYA
NAGHVESQSALFMRDNGISEGLVFHNNPEGTCGFCVNMTETLLPENAKMTVVPPEG
VIPVKRGATGETKVFTGNSNGPKSPTKGGC
GSAG Variant 
GSYALGPYQISAPQLPAYNGQTVGTFYYVNDAGGLEGKVFSSGGPTPYPNYA
NAGHVESQSALFMRDNGISEGLVFHNNPEGTCGFCVNMTETLLPENAKMTVVPPEG
AIPVKRGATGETKVFTGNSNGPKSPTKGGC
GSVS Variant

GSYALGPYQISAPQLPAYNGQTVGTFYYVNDAGGLEGKVFSSGGPTPYPNYA
NAGHVESQSALFMRDNGISEGLVFHNNPEGTCGFCVNMTETLLPENAKMTVVPPEG
VIPVKRGATGETKVFTGNSNSPKSPTKGGC

An adenine deaminase that may be used in the base editor according to the present invention means an amino group deaminase capable of converting an adenine base into inosine, and may be derived from or mutated (e.g., engineered or evolved) from any organism (e.g., a eukaryote or prokaryote), including but not limited to algae, bacteria, fungi, plants, invertebrates, and mammals, for example, *E. coli, S. aureus, S. typhi, S. putrefaciens, H. influenzae, or C. crescentus.* Such adenine deaminase may be, for example, APOBEC, AID, or TadA, or a variant thereof. The aforementioned TadA may be, for example, TadA8e (SEQ ID NO: 1) or a truncated form or a variant thereof (for example, a variant improved or evolved to be applicable to deoxynucleotides). The above-mentioned variant of TadA8e may be, for example, one in which one or more of amino acid residues 23, 28, 30, 36, 46, 48, 49, 51, 76, 82, 82, 84, 106, 108, 110, 111, 146, 147, 152, 154, 155, 156 and 157 of SEQ ID NO: 1 are mutated to another amino acid. Regarding the composition of adenine deaminase that may be used in the present invention, reference may be made to international patent application publications WO 2022/060185, WO 2023/086953, and the like, which are incorporated by reference in their entirety into this application, and which were already known prior to the present application. An adenine deaminase that may be used in the present invention may comprise the amino acid sequence of SEQ ID NO: 1 or a conservative amino acid substitution thereof.

The DNA binding protein used in the base editor according to the present invention may be a zinc finger protein, a TALE protein, a CRISPR-associated nuclease, or a combination thereof. With respect to the compositions of the zinc finger protein, the TALE protein, and the CRISPR-associated nuclease, reference may be made to prior art known before the filing of the present application, including International Patent Application Publication No. WO2022/060185, which is incorporated by reference in its entirety into this application.

The zinc finger is a representative DNA-binding protein structure that forms a major DNA-binding protein motif, and the interaction between the α-helix of the zinc finger and the major groove of DNA enables strong and specific recognition of DNA sequences. When one or more zinc finger motifs are used in combination and a DNA binding protein used in a base editor according to the present invention is a zinc finger protein, amino acid sequences of SEQ ID NOs: 7 to 9 may be included.

The DNA binding protein used in the base editor according to the present invention may be a TALE protein. The TALE protein of the present invention refers to a protein that binds to nucleotides in a sequence-specific manner via one or more TALE-repeat modules. The TALE protein comprises at least one TALE-repeat module, preferably, but not limited to, 1 to 30 TALE-repeat modules. As used herein, the TALE-repeat modules may be referred to as a "TALE array", and the term "TALE protein" refers to a configuration comprising an N-terminal domain and a C-terminal domain (which may comprise a half domain) on each side of the TALE array. The term "TALE" as used herein may mean only "TALE array" or "TALE protein" depending on the context. When the DNA binding protein used in the base editor according to the present invention is a TALE protein, amino acid sequences of SEQ ID NOs: 10 to 65 may be included.

When a TALE protein is used as a DNA binding protein of a base editor used in the present invention, a single module TALE array or a multi-module TALE array (e.g., a dual module TALE array comprising a first TALE (or left TALE) array and a second TALE (or right TALE) array) may be used.

When the base editor used in the present invention comprises two fusion proteins, each of which comprises a TALE protein, the two fusion proteins respectively have a first TALE protein (or left TALE) and a second TALE protein (or right TALE). The first TALE protein and the second TALE protein may each independently be linked, directly or indirectly (e.g., via a linker and/or other protein component), to at least one of a cytosine deaminase and an adenine deaminase. For example, a first fusion protein (a fusion protein that binds DNA 5' upstream from the base-editing target site)comprising a first TALE protein (left TALE) may comprise a first split of cytosine deaminase, a second fusion protein comprising a second TALE protein (right TALE) may comprise a second split of cytosine deaminase, and either or both of the first fusion protein and the second fusion protein may comprise an adenine deaminase. Alternatively, a first fusion protein comprising a first TALE protein (left TALE) may comprise a second split of cytosine deaminase, a second fusion protein comprising a second TALE protein (right TALE) may comprise a first split of cytosine deaminase, and either or both of the first fusion protein and the second fusion protein may comprise an adenine deaminase. Alternatively, the full-length form of cytosine deaminase may be included in either a first fusion protein comprising a first TALE protein or a second fusion protein comprising a second TALE protein, and either or both of the first fusion protein and the second fusion protein may comprise an adenine deaminase.

When the base editor used in the present invention comprises two fusion proteins, one of the two fusion proteins may comprise a TALE protein and the other may comprise a zinc finger protein. For example, a first fusion protein (a fusion protein that binds DNA 5' upstream from a base-editing target site) may comprise a TALE protein (left TALE), and a second fusion protein (a fusion protein that binds DNA 3' downstream from the base-editing target site) may comprise a zinc finger protein (right ZF). Alternatively, a first fusion protein (a fusion protein that binds DNA 5' upstream from a base-editing target site) may comprise a zinc finger protein (left ZF), and a second fusion protein (a fusion protein that binds DNA 3' downstream from the base-editing target site) may comprise a TALE protein (right TALE). In both of the above two methods, either or both of the first fusion protein and the second fusion protein may comprise an adenine deaminase.

When a cytosine deaminase is included in a fusion protein comprising a TALE protein or a zinc finger protein, the cytosine deaminase may be linked directly or indirectly (e.g., via a linker and/or other protein component) to the N-terminus or C-terminus (preferably the C-terminus) of the TALE protein, or to the N-terminus or C-terminus (preferably the N-terminus) of the zinc finger protein. When an adenine deaminase is included in a fusion protein comprising a TALE protein or a zinc finger protein, the adenine deaminase may be directly or indirectly linked (e.g., via a linker and/or other protein component) to the N-terminus or C-terminus (preferably the C-terminus) of the TALE protein or to the N-terminus or C-terminus (preferably the N-terminus) of the zinc finger protein. When both of the a cytosine deaminase and an adenine deaminase are included in a fusion protein comprising a TALE protein or a zinc finger protein, the adenine deaminase may be linked directly or indirectly (e.g., via a linker and/or other protein component) to the N-terminus or C-terminus (preferably the C-terminus) of the cytosine deaminase.

When the base editor according to the present invention comprises two fusion proteins, the fusion proteins may have different combinations and arrangements of protein components (e.g., DNA binding protein, cytosine deaminase, and adenine deaminase). For example, while a first fusion protein (a fusion protein that binds DNA 5' upstream from a base-editing target site) may comprise an adenine deaminase, a second fusion protein (a fusion protein that binds DNA 3' downstream from the base-editing target site) may not comprise an adenine deaminase, or vice versa. For example, while a first fusion protein (a fusion protein that binds DNA 5' upstream from a base-editing target site) may comprise a TALE protein, a second fusion protein (a fusion protein that binds DNA 3' downstream from the base-editing target site) may comprise a zinc finger protein, or vice versa.

When it comes to the arrangement of protein components of the fusion proteins, a first fusion protein (a fusion protein that binds to DNA 5' upstream from the base-editing target site) and a second fusion protein (a fusion protein that binds to DNA 3' downstream from the base-editing target site) may have independently arranged protein components. For example, in the first fusion protein, the adenine deaminase may be positioned after the C-terminus of the cytosine deaminase, whereas in the second fusion protein, the adenine deaminase may be positioned before the N-terminus of the cytosine deaminase, or vice versa. For example, in the first fusion protein, the DNA binding protein may be positioned after the C-terminus of the cytosine deaminase, whereas in the second fusion protein, the DNA binding protein may be positioned before the N-terminus of the cytosine deaminase, or vice versa.

One or more fusion proteins included in the base editor according to the present invention may each independently additionally include a UGI (uracil glycosylase inhibitor). UGI may increase base editing efficiency by inhibiting the activity of UDG (uracil DNA glycosylase), which is an enzyme that repairs mutated DNA by catalyzing the removal of U from DNA. When a UGI is used in the base editor according to the present invention, the location of the UGI may vary, and for example, the UGI may be directly or indirectly linked (for example, via a linker and/or other protein components) to the C-terminus of cytosine deaminase, but is not limited thereto. When UGI is used in the base editor according to the present invention, the UGI may comprise the amino acid sequence of SEQ ID NO: 126.

One or more fusion proteins included in the base editor according to the present invention may additionally include a nuclear export signal (NES). Attaching an NES to a base-editing protein may result in higher efficiency of base editing. The NES sequence may be any signal sequence (e.g., SEQ ID NO: 127) that confers the ability to translocate outside the nucleus, and a natural NES or an artificially synthesized NES may be used. For example, it may be derived from mirute virus of mice (MVM), but is not limited to,. When an NES is used in the base editor according to the present invention, the location of the NES may vary and may be, for example, directly or indirectly linked to the N-terminus of cytosine deaminase (e.g., via a linker and/or other protein components), but is not limited to. When an NES is used in the base editor according to the present invention, the NES may comprise the amino acid sequence of SEQ ID NO: 127.

The base editor (fusion protein) according to the present invention may additionally include an MTS (mitochondrial targeting sequence). The MTS may be any signal sequence capable of translocating into mitochondria, and may be a natural MTS present at the N-terminus of various mitochondrial proteins, or an artificially synthesized MTS may also be used. When MTS is used in the base editor according to the present invention, the location of the MTS may vary, and for example, the MTS may be directly or indirectly linked (for example, via a linker and/or other protein components) to the N-terminus of a DNA binding protein or the N-terminus of an NES, but is not limited thereto. When MTS is used in the base editor according to the present invention, the MTS may comprise any one of the amino acid sequences of SEQ ID NOs: 128 to 130.

The DNA binding protein used in the base editor according to the present invention, in whole or in part, may recognize and bind to the nucleotide sequence of 5'-TACGGGCTACTACAACCCTTCGCTGAC**A**CCATAAAACTCTTCACCAAAGAGCCCCT AAA-3' (the underlined and bold A is the base at position 3460) or a portion thereof of the mitochondrial ND1 DNA sequence. Preferably, it may recognize 5'-TACGGGCTACTACAACCCTTCG-3' or a portion thereof, and/or 5'-TAAAACTCTTCACCAAAGAGCCCCTAAA-3' or a portion thereof of the mitochondrial ND1 DNA sequence. When the base editor according to the present invention is in the form of a composition of one or more different base editors, one base editor (the first fusion protein) may recognize 5'- TACGGGCTACTACAACCCTTCG-3' or a portion thereof of the mitochondrial DNA sequence, and another base editor (the second fusion protein) may recognize 5'- TAAAACTCTTCACCAAAGAGCCCCTAAA-3' or a portion thereof of the mitochondrial DNA sequence.

The DNA binding protein used in the base editor according to the present invention, in whole or in part, may recognize and bind to the nucleotide sequence of 5'-CAAACTCAAACTACGAACGCACTCACAGTCACATCATAATCCTCTCTCAAGGACTT CAAAC-3' (the underlined and bold A is the base at position 11778) or a portion thereof of the mitochondrial ND4 DNA sequence. Preferably, it may recognize 5'-CAAACTCAAACTACGAACGCACTCACAGTC-3' or a portion thereof, and/or 5'-CATAATCCTCTCTCAAGGACTTCAAAC-3' or a portion thereof of the mitochondrial ND4 DNA sequence. When the base editor according to the present invention is in the form of a composition of one or more different base editors, one base editor (the first fusion protein) may recognize 5'- CAAACTCAAACTACGAACGCACTCACAGTC-3' or a portion thereof of the mitochondrial DNA sequence, and another base editor (the second fusion protein) may recognize 5'- CATAATCCTCTCTCAAGGACTTCAAAC-3' or a portion thereof of the mitochondrial DNA sequence.

The DNA binding protein used in the base editor according to the present invention, in whole or in part, may recognize 5'-TAGCCATCGCTGTAGTATATCCAAAGACAACCA**C**CATTCCCCCTAAATAAATTAAAA AAACTA-3' (the underlined and bold C is the base at position 14484) in a mitochondrial ND6 DNA sequence or a portion thereof, preferably 5'-TCGCTGTAGTATATCCAAAGACAACCACCATTCCCCCTAAATAAATTAAAAAAACT A-3' or a portion thereof. Preferably, it may recognize 5'-TCGCTGTAGTATATCCAAAGACA-3' or a portion thereof, and/or 5'-TCCCCCTAAATAAATTAAAAA-3' or a portion thereof of the mitochondrial DNA sequence. When the base editor according to the present invention is in the form of a composition of one or more different base editors, one base editor (the first fusion protein) may recognize 5'-TCGCTGTAGTATATCCAAAGACA-3' or a portion thereof of the mitochondrial DNA sequence, and another base editor (the second fusion protein) may recognize 5'-TCCCCCTAAATAAATTAAAAA-3' or a portion thereof of the mitochondrial DNA sequence.

The DNA binding protein used in the base editor according to the present invention may comprise any one of the amino acid sequences of SEQ ID NOs: 7 to 65, or a conservative substitution thereof.

A "conservative amino acid substitution" refers to the substitution of an amino acid residue with another residue having a side chain (R group) with similar chemical properties (e.g., charge or hydrophobicity). In general, conservative amino acid substitutions do not substantially change the functional properties of a protein. When two or more amino acid sequences differ from each other by conservative substitutions, the % sequence identity or similarity may be adjusted upward to compensate for the conservative nature of the substitutions. Means for performing this adjustment are well known to those skilled in the art [see Pearson (1994) Methods Mol. Biol. 24: 307-31]. Examples of amino acid groups having side chains with similar chemical properties include: (1) an aliphatic side chain: glycine, alanine, valine, leucine, and isoleucine; (2) an aliphatic-hydroxyl side chain: serine and threonine; (3) an amide-containing side chain: asparagine and glutamine; (4) an aromatic side chain: phenylalanine, tyrosine, and tryptophan; (5) a basic side chain: lysine, arginine, and histidine; (6) an acidic side chain: aspartate and glutamate; and (7) a sulfur-containing side chain: cysteine and methionine. Preferred conservative amino acid substitution groups are: valine-leucine-isoleucine, phenylalanine-tyrosine, lysine-arginine, alanine-valine, glutamate-aspartate, and asparagine-glutamine. Alternatively, a conservative substitution may be any change having a positive value in the PAM250 log-odds matrix described in Gonnet et al. (1992) Science 256: 1443-1445.

The base editing composition of the present invention is capable of editing adenine (A) at position 3460 of mitochondrial ND1 DNA to guanine (G) in a patient with LHON, and comprises two fusion proteins, wherein the two fusion proteins each comprise a TALE protein and a DddAₜₒₓ split that specifically bind to mitochondrial ND1 DNA, and one of the two fusion proteins may additionally comprise TadA8e.

In the base editing composition, one fusion protein may comprise a TALE protein (left TALE) comprising an amino acid sequence selected from the group consisting of SEQ ID NOs: 10 to 17 or a conservative amino acid substitution thereof, and the other fusion protein may comprise a TALE protein (right TALE) comprising an amino acid sequence selected from the group consisting of SEQ ID NOs: 18 to 31 or a conservative amino acid substitution thereof.

The base editing composition of the present invention is capable of editing adenine (A) at position 11778 of mitochondrial ND4 DNA to guanine (G) in a patient with LHON, and comprises two fusion proteins, wherein the two fusion proteins each comprise a TALE or zinc finger protein and a DddAₜₒₓ split that specifically bind to mitochondrial ND4 DNA, and one of the two fusion proteins may further comprise TadA8e.

In the base editing composition, one fusion protein may comprise a TALE protein (left TALE) comprising an amino acid sequence selected from the group consisting of SEQ ID NOs: 32 to 42 or a conservative amino acid substitution thereof, or a zinc finger protein (left ZF) comprising an amino acid sequence selected from the group consisting of SEQ ID NOs: 7 to 9 or a conservative amino acid substitution thereof, and the other fusion protein may comprise a TALE protein (right TALE) comprising an amino acid sequence selected from the group consisting of SEQ ID NOs: 43 to 53 or a conservative amino acid substitution thereof, or a zinc finger protein (right ZF) comprising an amino acid sequence selected from the group consisting of SEQ ID NOs: 7 to 9 or a conservative amino acid substitution thereof.

The base editing composition of the present invention is capable of editing cytosine (A) at position 14484 of mitochondrial ND6 DNA to thymine (T) in a patient with LHON, and comprises two fusion proteins, wherein the two fusion proteins may each include a TALE protein and a DddAₜₒₓ split that specifically bind to mitochondrial ND6 DNA.

In the base editing composition, one fusion protein may comprise a TALE protein (left TALE) comprising an amino acid sequence selected from the group consisting of SEQ ID NOs: 54 to 57 or a conservative amino acid substitution thereof, and the other fusion protein may comprise a TALE protein (right TALE) comprising an amino acid sequence selected from the group consisting of SEQ ID NOs: 58 to 65 or a conservative amino acid substitution thereof.

The term "fusion protein" as used herein refers to a polypeptide formed by binding two or more different polypeptides via peptide bonds. The fusion protein according to the present invention is capable of editing adenine (A) at position 3460 of mitochondrial DNA in a patient with LHON to guanine (G), adenine (A) at position 11778 to guanine (G), or cytosine (A) at position 14484 to thymine (T), and comprises a DNA binding protein, additionally comprises a deaminase (at least one of adenine deaminase and cytosine deaminase), and may further comprise UGI, NES, and/or MTS. A method for designing and constructing a fusion protein (or a polynucleotide encoding a fusion protein) may be any method known in the art, and the polynucleotide may be inserted into a vector, and the vector may be introduced into a cell. Individual proteins constituting a fusion protein according to the present invention are typically cloned into a single polynucleotide and expressed as a single polypeptide (fusion protein), but one or more of the individual proteins may be cloned into separate polynucleotides and expressed as two or more separate polypeptides, and such a case also falls within the scope of the present invention.

A linker that may be used in a fusion protein according to the present invention may be a peptide linker comprising 2 to 40 amino acid residues. The length may be, for example, a length of 2, 5, 10, 16, 24, or 32 amino acids, but is not limited thereto. Linkers used in the present invention may comprise, for example, the following.
GS
SGSETPGTSESATPES (SEQ ID NO: 131)
SGTPHEVGVYTLSGTPHEVGVYTL (SEQ ID NO: 132)
AAEFGIHGVPAAMG (SEQ ID NO: 133)
AAEFGIHGVPAAMGGS (SEQ ID NO: 134)
SGGS (SEQ ID NO: 135)

The present invention provides a polynucleotide encoding any one of one or more fusion proteins included in the base editing composition. A base editor according to the present invention may comprise a polynucleotide encoding the fusion protein described above. The base editor according to the present invention may be in the form of a composition of different polynucleotides encoding different base editors.

### Composition Examples Of Base Editor (Or Base Editing Composition) According To The Present Invention

The base editor (or base editing composition) according to the present invention may comprise a combination of a fusion protein having an amino acid sequence selected from the group consisting of SEQ ID NOs: 78 to 85 and a fusion protein having an amino acid sequence selected from the group consisting of SEQ ID NOs: 86 to 99.

For example, a base editor (or base editing composition) according to the present invention may comprise a combination of fusion proteins selected from the group consisting of the following pairs of fusion proteins:
A fusion protein having an amino acid sequence of SEQ ID NO: 78 and a fusion protein having an amino acid sequence of SEQ ID NO: 90;
A fusion protein having an amino acid sequence of SEQ ID NO: 78 and a fusion protein having an amino acid sequence of SEQ ID NO: 91;
A fusion protein having an amino acid sequence of SEQ ID NO: 79 and a fusion protein having an amino acid sequence of SEQ ID NO: 92;
A fusion protein having an amino acid sequence of SEQ ID NO: 79 and a fusion protein having an amino acid sequence of SEQ ID NO: 93;
A fusion protein having an amino acid sequence of SEQ ID NO: 79 and a fusion protein having an amino acid sequence of SEQ ID NO: 94;
A fusion protein having an amino acid sequence of SEQ ID NO: 79 and a fusion protein having an amino acid sequence of SEQ ID NO: 90;
fusion protein having an amino acid sequence of SEQ ID NO: 79 and a fusion protein having an amino acid sequence of SEQ ID NO: 91;
A fusion protein having an amino acid sequence of SEQ ID NO: 80 and a fusion protein having an amino acid sequence of SEQ ID NO: 95;
A fusion protein having an amino acid sequence of SEQ ID NO: 80 and a fusion protein having an amino acid sequence of SEQ ID NO: 90;
A fusion protein having an amino acid sequence of SEQ ID NO: 80 and a fusion protein having an amino acid sequence of SEQ ID NO: 91;
A fusion protein having an amino acid sequence of SEQ ID NO: 81 and a fusion protein having an amino acid sequence of SEQ ID NO: 96;
A fusion protein having an amino acid sequence of SEQ ID NO: 81 and a fusion protein having an amino acid sequence of SEQ ID NO: 91;
A fusion protein having an amino acid sequence of SEQ ID NO: 82 and a fusion protein having an amino acid sequence of SEQ ID NO: 97;
A fusion protein having an amino acid sequence of SEQ ID NO: 82 and a fusion protein having an amino acid sequence of SEQ ID NO: 90;
A fusion protein having an amino acid sequence of SEQ ID NO: 83 and a fusion protein having an amino acid sequence of SEQ ID NO: 88;
A fusion protein having an amino acid sequence of SEQ ID NO: 83 and a fusion protein having an amino acid sequence of SEQ ID NO: 87;
A fusion protein having an amino acid sequence of SEQ ID NO: 83 and a fusion protein having an amino acid sequence of SEQ ID NO: 89;
A fusion protein having an amino acid sequence of SEQ ID NO: 84 and a fusion protein having an amino acid sequence of SEQ ID NO: 88;
A fusion protein having an amino acid sequence of SEQ ID NO: 84 and a fusion protein having an amino acid sequence of SEQ ID NO: 86;
A fusion protein having an amino acid sequence of SEQ ID NO: 84 and a fusion protein having an amino acid sequence of SEQ ID NO: 87; and
A fusion protein having an amino acid sequence of SEQ ID NO: 85 and a fusion protein having an amino acid sequence of SEQ ID NO: 88.

The base editor (or base editing composition) according to the present invention may comprise a combination of a fusion protein comprising an amino acid sequence selected from the group consisting of SEQ ID NOs: 100 to 114 and a fusion protein comprising an amino acid sequence selected from the group consisting of SEQ ID NOs: 115 to 125.

For example, a base editor (or base editing composition) according to the present invention may comprise a combination of fusion proteins selected from the group consisting of the following pairs of fusion proteins:
A fusion protein having an amino acid sequence of SEQ ID NO: 100 and a fusion protein having an amino acid sequence of SEQ ID NO: 118;
A fusion protein having an amino acid sequence of SEQ ID NO: 101 and a fusion protein having an amino acid sequence of SEQ ID NO: 119;
A fusion protein having an amino acid sequence of SEQ ID NO: 101 and a fusion protein having an amino acid sequence of SEQ ID NO: 118;
A fusion protein having an amino acid sequence of SEQ ID NO: 102 and a fusion protein having an amino acid sequence of SEQ ID NO: 118;
fusion protein having an amino acid sequence of SEQ ID NO: 103 and a fusion protein having an amino acid sequence of SEQ ID NO: 120;
A fusion protein having an amino acid sequence of SEQ ID NO: 103 and a fusion protein having an amino acid sequence of SEQ ID NO: 119;
A fusion protein having an amino acid sequence of SEQ ID NO: 103 and a fusion protein having an amino acid sequence of SEQ ID NO: 118;
A fusion protein having an amino acid sequence of SEQ ID NO: 113 and a fusion protein having an amino acid sequence of SEQ ID NO: 118;
A fusion protein having an amino acid sequence of SEQ ID NO: 104 and a fusion protein having an amino acid sequence of SEQ ID NO: 119;
A fusion protein having an amino acid sequence of SEQ ID NO: 104 and a fusion protein having an amino acid sequence of SEQ ID NO: 118;
A fusion protein having an amino acid sequence of SEQ ID NO: 104 and a fusion protein having an amino acid sequence of SEQ ID NO: 121;
A fusion protein having an amino acid sequence of SEQ ID NO: 104 and a fusion protein having an amino acid sequence of SEQ ID NO: 122;
A fusion protein having an amino acid sequence of SEQ ID NO: 105 and a fusion protein having an amino acid sequence of SEQ ID NO: 120;
A fusion protein having an amino acid sequence of SEQ ID NO: 105 and a fusion protein having an amino acid sequence of SEQ ID NO: 123;
A fusion protein having an amino acid sequence of SEQ ID NO: 105 and a fusion protein having an amino acid sequence of SEQ ID NO: 119;
A fusion protein having an amino acid sequence of SEQ ID NO: 105 and a fusion protein having an amino acid sequence of SEQ ID NO: 118;
A fusion protein having an amino acid sequence of SEQ ID NO: 105 and a fusion protein having an amino acid sequence of SEQ ID NO: 121;
A fusion protein having an amino acid sequence of SEQ ID NO: 105 and a fusion protein having an amino acid sequence of SEQ ID NO: 122;
A fusion protein having an amino acid sequence of SEQ ID NO: 106 and a fusion protein having an amino acid sequence of SEQ ID NO: 119;
A fusion protein having an amino acid sequence of SEQ ID NO: 106 and a fusion protein having an amino acid sequence of SEQ ID NO: 118;
A fusion protein having an amino acid sequence of SEQ ID NO: 106 and a fusion protein having an amino acid sequence of SEQ ID NO: 121;
A fusion protein having an amino acid sequence of SEQ ID NO: 106 and a fusion protein having an amino acid sequence of SEQ ID NO: 122;
A fusion protein having an amino acid sequence of SEQ ID NO: 106 and a fusion protein having an amino acid sequence of SEQ ID NO: 124;
A fusion protein having an amino acid sequence of SEQ ID NO: 111 and a fusion protein having an amino acid sequence of SEQ ID NO: 120;
A fusion protein having an amino acid sequence of SEQ ID NO: 111 and a fusion protein having an amino acid sequence of SEQ ID NO: 118;
fusion protein having an amino acid sequence of SEQ ID NO: 107 and a fusion protein having an amino acid sequence of SEQ ID NO: 118;
A fusion protein having an amino acid sequence of SEQ ID NO: 108 and a fusion protein having an amino acid sequence of SEQ ID NO: 120;
A fusion protein having an amino acid sequence of SEQ ID NO: 108 and a fusion protein having an amino acid sequence of SEQ ID NO: 119;
A fusion protein having an amino acid sequence of SEQ ID NO: 108 and a fusion protein having an amino acid sequence of SEQ ID NO: 118;
A fusion protein having an amino acid sequence of SEQ ID NO: 108 and a fusion protein having an amino acid sequence of SEQ ID NO: 121;
A fusion protein having an amino acid sequence of SEQ ID NO: 108 and a fusion protein having an amino acid sequence of SEQ ID NO: 122;
A fusion protein having an amino acid sequence of SEQ ID NO: 108 and a fusion protein having an amino acid sequence of SEQ ID NO: 124;
A fusion protein having an amino acid sequence of SEQ ID NO: 112 and a fusion protein having an amino acid sequence of SEQ ID NO: 120;
A fusion protein having an amino acid sequence of SEQ ID NO: 112 and a fusion protein having an amino acid sequence of SEQ ID NO: 118;
A fusion protein having an amino acid sequence of SEQ ID NO: 112 and a fusion protein having an amino acid sequence of SEQ ID NO: 121;
A fusion protein having an amino acid sequence of SEQ ID NO: 112 and a fusion protein having an amino acid sequence of SEQ ID NO: 124;
A fusion protein having an amino acid sequence of SEQ ID NO: 114 and a fusion protein having an amino acid sequence of SEQ ID NO: 115;
A fusion protein having an amino acid sequence of SEQ ID NO: 114 and a fusion protein having an amino acid sequence of SEQ ID NO: 116;
A fusion protein having an amino acid sequence of SEQ ID NO: 109 and a fusion protein having an amino acid sequence of SEQ ID NO: 117;
A fusion protein having an amino acid sequence of SEQ ID NO: 109 and a fusion protein having an amino acid sequence of SEQ ID NO: 115;
A fusion protein having an amino acid sequence of SEQ ID NO: 109 and a fusion protein having an amino acid sequence of SEQ ID NO: 116; and
A fusion protein having an amino acid sequence of SEQ ID NO: 110 and a fusion protein having an amino acid sequence of SEQ ID NO: 115.

The base editor (or base editing composition) according to the present invention may comprise a combination of a fusion protein having an amino acid sequence selected from the group consisting of SEQ ID NOs: 66 to 69 and a fusion protein having an amino acid sequence selected from the group consisting of SEQ ID NOs: 70 to 77.

For example, a base editor (or base editing composition) according to the present invention may comprise a combination of fusion proteins selected from the group consisting of f the following pairs of fusion proteins:
A fusion protein having an amino acid sequence of SEQ ID NO: 66 and a fusion protein having an amino acid sequence of SEQ ID NO: 70;
A fusion protein having an amino acid sequence of SEQ ID NO: 67 and a fusion protein having an amino acid sequence of SEQ ID NO: 70;
A fusion protein having an amino acid sequence of SEQ ID NO: 66 and a fusion protein having an amino acid sequence of SEQ ID NO: 71;
A fusion protein having an amino acid sequence of SEQ ID NO: 67 and a fusion protein having an amino acid sequence of SEQ ID NO: 71;
A fusion protein having an amino acid sequence of SEQ ID NO: 66 and a fusion protein having an amino acid sequence of SEQ ID NO: 72;
A fusion protein having an amino acid sequence of SEQ ID NO: 66 and a fusion protein having an amino acid sequence of SEQ ID NO: 73;
A fusion protein having an amino acid sequence of SEQ ID NO: 67 and a fusion protein having an amino acid sequence of SEQ ID NO: 72;
A fusion protein having an amino acid sequence of SEQ ID NO: 67 and a fusion protein having an amino acid sequence of SEQ ID NO: 73;
A fusion protein having an amino acid sequence of SEQ ID NO: 68 and a fusion protein having an amino acid sequence of SEQ ID NO: 74;
A fusion protein having an amino acid sequence of SEQ ID NO: 68 and a fusion protein having an amino acid sequence of SEQ ID NO: 75;
A fusion protein having an amino acid sequence of SEQ ID NO: 68 and a fusion protein having an amino acid sequence of SEQ ID NO: 76;
A fusion protein having an amino acid sequence of SEQ ID NO: 68 and a fusion protein having an amino acid sequence of SEQ ID NO: 77;
A fusion protein having an amino acid sequence of SEQ ID NO: 69 and a fusion protein having an amino acid sequence of SEQ ID NO: 74;
A fusion protein having an amino acid sequence of SEQ ID NO: 69 and a fusion protein having an amino acid sequence of SEQ ID NO: 75; and
A fusion protein having an amino acid sequence of SEQ ID NO: 69 and a fusion protein having an amino acid sequence of SEQ ID NO: 76.

The base editor (or base editing composition) according to the present invention may comprise a combination of polynucleotides comprising a polynucleotide encoding an amino acid sequence selected from the group consisting of SEQ ID NOs: 78 to 85 and a polynucleotide encoding an amino acid sequence selected from the group consisting of SEQ ID NOs: 86 to 99.

For example, the base editor (or base editing composition) according to the present invention may comprise a combination of polynucleotides selected from the group consisting of the following pairs of polynucleotide sequences:
A polynucleotide encoding an amino acid sequence of SEQ ID NO: 78 and a polynucleotide encoding an amino acid sequence of SEQ ID NO: 90;
A polynucleotide encoding an amino acid sequence of SEQ ID NO: 78 and a polynucleotide encoding an amino acid sequence of SEQ ID NO: 91;
A polynucleotide encoding an amino acid sequence of SEQ ID NO: 79 and a polynucleotide encoding an amino acid sequence of SEQ ID NO: 92;
A polynucleotide encoding an amino acid sequence of SEQ ID NO: 79 and a polynucleotide encoding an amino acid sequence of SEQ ID NO: 93;
A polynucleotide encoding an amino acid sequence of SEQ ID NO: 79 and a polynucleotide encoding an amino acid sequence of SEQ ID NO: 94;
A polynucleotide encoding an amino acid sequence of SEQ ID NO: 79 and a polynucleotide encoding an amino acid sequence of SEQ ID NO: 90;
A polynucleotide encoding an amino acid sequence of SEQ ID NO: 79 and a polynucleotide encoding an amino acid sequence of SEQ ID NO: 91;
A polynucleotide encoding an amino acid sequence of SEQ ID NO: 80 and a polynucleotide encoding an amino acid sequence of SEQ ID NO: 95;
A polynucleotide encoding an amino acid sequence of SEQ ID NO: 80 and a polynucleotide encoding an amino acid sequence of SEQ ID NO: 90;
A polynucleotide encoding an amino acid sequence of SEQ ID NO: 80 and a polynucleotide encoding an amino acid sequence of SEQ ID NO: 91;
A polynucleotide encoding an amino acid sequence of SEQ ID NO: 81 and a polynucleotide encoding an amino acid sequence of SEQ ID NO: 96;
A polynucleotide encoding an amino acid sequence of SEQ ID NO: 81 and a polynucleotide encoding an amino acid sequence of SEQ ID NO: 91;
A polynucleotide encoding an amino acid sequence of SEQ ID NO: 82 and a polynucleotide encoding an amino acid sequence of SEQ ID NO: 97;
A polynucleotide encoding an amino acid sequence of SEQ ID NO: 82 and a polynucleotide encoding an amino acid sequence of SEQ ID NO: 90;
A polynucleotide encoding an amino acid sequence of SEQ ID NO: 83 and a polynucleotide encoding an amino acid sequence of SEQ ID NO: 88;
A polynucleotide encoding an amino acid sequence of SEQ ID NO: 83 and a polynucleotide encoding an amino acid sequence of SEQ ID NO: 87;
A polynucleotide encoding an amino acid sequence of SEQ ID NO: 83 and a polynucleotide encoding an amino acid sequence of SEQ ID NO: 89;
A polynucleotide encoding an amino acid sequence of SEQ ID NO: 84 and a polynucleotide encoding an amino acid sequence of SEQ ID NO: 88;
A polynucleotide encoding an amino acid sequence of SEQ ID NO: 84 and a polynucleotide encoding an amino acid sequence of SEQ ID NO: 86;
A polynucleotide encoding an amino acid sequence of SEQ ID NO: 84 and a polynucleotide encoding an amino acid sequence of SEQ ID NO: 87; and
A polynucleotide encoding an amino acid sequence of SEQ ID NO: 85 and a polynucleotide encoding an amino acid sequence of SEQ ID NO: 88.

The base editor (or base editing composition) according to the present invention may comprise a combination of a polynucleotide encoding an amino acid sequence selected from the group consisting of SEQ ID NOs: 100 to 114 and a polynucleotide encoding an amino acid sequence selected from the group consisting of SEQ ID NOs: 115 to 125.

For example, the base editor (or base editing composition) according to the present invention may comprise a combination of polynucleotides selected from the group consisting of the following pairs of polynucleotide sequences:
A polynucleotide encoding an amino acid sequence of SEQ ID NO: 100 and a polynucleotide encoding an amino acid sequence of SEQ ID NO: 118;
A polynucleotide encoding an amino acid sequence of SEQ ID NO: 101 and a polynucleotide encoding an amino acid sequence of SEQ ID NO: 119;
A polynucleotide encoding an amino acid sequence of SEQ ID NO: 101 and a polynucleotide encoding an amino acid sequence of SEQ ID NO: 118;
A polynucleotide encoding an amino acid sequence of SEQ ID NO: 102 and a polynucleotide encoding an amino acid sequence of SEQ ID NO: 118;
A polynucleotide encoding an amino acid sequence of SEQ ID NO: 103 and a polynucleotide encoding an amino acid sequence of SEQ ID NO: 120;
A polynucleotide encoding an amino acid sequence of SEQ ID NO: 103 and a polynucleotide encoding an amino acid sequence of SEQ ID NO: 119;
A polynucleotide encoding an amino acid sequence of SEQ ID NO: 103 and a polynucleotide encoding an amino acid sequence of SEQ ID NO: 118;
A polynucleotide encoding an amino acid sequence of SEQ ID NO: 113 and a polynucleotide encoding an amino acid sequence of SEQ ID NO: 118;
A polynucleotide encoding an amino acid sequence of SEQ ID NO: 104 and a polynucleotide encoding an amino acid sequence of SEQ ID NO: 119;
A polynucleotide encoding an amino acid sequence of SEQ ID NO: 104 and a polynucleotide encoding an amino acid sequence of SEQ ID NO: 118;
A polynucleotide encoding an amino acid sequence of SEQ ID NO: 104 and a polynucleotide encoding an amino acid sequence of SEQ ID NO: 121;
A polynucleotide encoding an amino acid sequence of SEQ ID NO: 104 and a polynucleotide encoding an amino acid sequence of SEQ ID NO: 122;
A polynucleotide encoding an amino acid sequence of SEQ ID NO: 105 and a polynucleotide encoding an amino acid sequence of SEQ ID NO: 120;
A polynucleotide encoding an amino acid sequence of SEQ ID NO: 105 and a polynucleotide encoding an amino acid sequence of SEQ ID NO: 123;
A polynucleotide encoding an amino acid sequence of SEQ ID NO: 105 and a polynucleotide encoding an amino acid sequence of SEQ ID NO: 119;
A polynucleotide encoding an amino acid sequence of SEQ ID NO: 105 and a polynucleotide encoding an amino acid sequence of SEQ ID NO: 118;
A polynucleotide encoding an amino acid sequence of SEQ ID NO: 105 and a polynucleotide encoding an amino acid sequence of SEQ ID NO: 121;
A polynucleotide encoding an amino acid sequence of SEQ ID NO: 105 and a polynucleotide encoding an amino acid sequence of SEQ ID NO: 122;
A polynucleotide encoding an amino acid sequence of SEQ ID NO: 106 and a polynucleotide encoding an amino acid sequence of SEQ ID NO: 119;
A polynucleotide encoding an amino acid sequence of SEQ ID NO: 106 and a polynucleotide encoding an amino acid sequence of SEQ ID NO: 118;
A polynucleotide encoding an amino acid sequence of SEQ ID NO: 106 and a polynucleotide encoding an amino acid sequence of SEQ ID NO: 121;
A polynucleotide encoding an amino acid sequence of SEQ ID NO: 106 and a polynucleotide encoding an amino acid sequence of SEQ ID NO: 122;
A polynucleotide encoding an amino acid sequence of SEQ ID NO: 106 and a polynucleotide encoding an amino acid sequence of SEQ ID NO: 124;
A polynucleotide encoding an amino acid sequence of SEQ ID NO: 111 and a polynucleotide encoding an amino acid sequence of SEQ ID NO: 120;
A polynucleotide encoding an amino acid sequence of SEQ ID NO: 111 and a polynucleotide encoding an amino acid sequence of SEQ ID NO: 118;
A polynucleotide encoding an amino acid sequence of SEQ ID NO: 107 and a polynucleotide encoding an amino acid sequence of SEQ ID NO: 118;
A polynucleotide encoding an amino acid sequence of SEQ ID NO: 108 and a polynucleotide encoding an amino acid sequence of SEQ ID NO: 120;
A polynucleotide encoding an amino acid sequence of SEQ ID NO: 108 and a polynucleotide encoding an amino acid sequence of SEQ ID NO: 119;
A polynucleotide encoding an amino acid sequence of SEQ ID NO: 108 and a polynucleotide encoding an amino acid sequence of SEQ ID NO: 118;
A polynucleotide encoding an amino acid sequence of SEQ ID NO: 108 and a polynucleotide encoding an amino acid sequence of SEQ ID NO: 121;
A polynucleotide encoding an amino acid sequence of SEQ ID NO: 108 and a polynucleotide encoding an amino acid sequence of SEQ ID NO: 122;
A polynucleotide encoding an amino acid sequence of SEQ ID NO: 108 and a polynucleotide encoding an amino acid sequence of SEQ ID NO: 124;
A polynucleotide encoding an amino acid sequence of SEQ ID NO: 112 and a polynucleotide encoding an amino acid sequence of SEQ ID NO: 120;
A polynucleotide encoding an amino acid sequence of SEQ ID NO: 112 and a polynucleotide encoding an amino acid sequence of SEQ ID NO: 118;
A polynucleotide encoding an amino acid sequence of SEQ ID NO: 112 and a polynucleotide encoding an amino acid sequence of SEQ ID NO: 121;
A polynucleotide encoding an amino acid sequence of SEQ ID NO: 112 and a polynucleotide encoding an amino acid sequence of SEQ ID NO: 124;
A polynucleotide encoding an amino acid sequence of SEQ ID NO: 114 and a polynucleotide encoding an amino acid sequence of SEQ ID NO: 115;
A polynucleotide encoding an amino acid sequence of SEQ ID NO: 114 and a polynucleotide encoding an amino acid sequence of SEQ ID NO: 116;
A polynucleotide encoding an amino acid sequence of SEQ ID NO: 109 and a polynucleotide encoding an amino acid sequence of SEQ ID NO: 117;
A polynucleotide encoding an amino acid sequence of SEQ ID NO: 109 and a polynucleotide encoding an amino acid sequence of SEQ ID NO: 115;
A polynucleotide encoding an amino acid sequence of SEQ ID NO: 109 and a polynucleotide encoding an amino acid sequence of SEQ ID NO: 116; and
A polynucleotide encoding an amino acid sequence of SEQ ID NO: 110 and a polynucleotide encoding an amino acid sequence of SEQ ID NO: 115.

The base editor (or base editing composition) according to the present invention may comprise a combination of a polynucleotide encoding an amino acid sequence selected from the group consisting of SEQ ID NOs: 66 to 69 and a polynucleotide encoding an amino acid sequence selected from the group consisting of SEQ ID NOs: 70 to 77.

For example, the base editor (or base editing composition) according to the present invention may comprise a combination of polynucleotides selected from the group consisting of the following pairs of polynucleotide sequences:
A polynucleotide encoding an amino acid sequence of SEQ ID NO: 66 and a polynucleotide encoding an amino acid sequence of SEQ ID NO: 70;
A polynucleotide encoding an amino acid sequence of SEQ ID NO: 67 and a polynucleotide encoding an amino acid sequence of SEQ ID NO: 70;
A polynucleotide encoding an amino acid sequence of SEQ ID NO: 66 and a polynucleotide encoding an amino acid sequence of SEQ ID NO: 71;
A polynucleotide encoding an amino acid sequence of SEQ ID NO: 67 and a polynucleotide encoding an amino acid sequence of SEQ ID NO: 71;
A polynucleotide encoding an amino acid sequence of SEQ ID NO: 66 and a polynucleotide encoding an amino acid sequence of SEQ ID NO: 72;
A polynucleotide encoding an amino acid sequence of SEQ ID NO: 66 and a polynucleotide encoding an amino acid sequence of SEQ ID NO: 73;
A polynucleotide encoding an amino acid sequence of SEQ ID NO: 67 and a polynucleotide encoding an amino acid sequence of SEQ ID NO: 72;
A polynucleotide encoding an amino acid sequence of SEQ ID NO: 67 and a polynucleotide encoding an amino acid sequence of SEQ ID NO: 73;
A polynucleotide encoding an amino acid sequence of SEQ ID NO: 68 and a polynucleotide encoding an amino acid sequence of SEQ ID NO: 74;
A polynucleotide encoding an amino acid sequence of SEQ ID NO: 68 and a polynucleotide encoding an amino acid sequence of SEQ ID NO: 75;
A polynucleotide encoding an amino acid sequence of SEQ ID NO: 68 and a polynucleotide encoding an amino acid sequence of SEQ ID NO: 76;
A polynucleotide encoding an amino acid sequence of SEQ ID NO: 68 and a polynucleotide encoding an amino acid sequence of SEQ ID NO: 77;
A polynucleotide encoding an amino acid sequence of SEQ ID NO: 69 and a polynucleotide encoding an amino acid sequence of SEQ ID NO: 74;
A polynucleotide encoding an amino acid sequence of SEQ ID NO: 69 and a polynucleotide encoding an amino acid sequence of SEQ ID NO: 75; and
A polynucleotide encoding an amino acid sequence of SEQ ID NO: 69 and a polynucleotide encoding an amino acid sequence of SEQ ID NO: 76.

The present invention provides a method of correcting a mitochondrial DNA base mutation in a patient with LHON, comprising contacting the mitochondrial DNA of the patient with a mitochondrial DNA base editor (or a base editing composition; for example, a combination of fusion proteins or polynucleotides described under the section "Composition Examples of Base Editor (or Base Editing Composition) According to the Present Invention"), wherein the correction involves correcting adenine (A) at position 3460 of mitochondrial ND1 DNA to guanine (G), or correcting adenine (A) at position 11778 of mitochondrial ND4 DNA to guanine (G), or correcting cytosine (A) at position 14484 of mitochondrial ND6 DNA to thymine (T).

The mitochondrial DNA base editor according to the present invention may correct adenine (A) at position 3460 of mitochondrial ND1 DNA to guanine (G), or adenine (A) at position 11778 of mitochondrial ND4 DNA to guanine (G), or cytosine (A) at position 14484 of mitochondrial ND6 DNA to thymine (T), with a frequency of 0.5% or more, 1% or more, 2% or more, 3% or more, 4% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, or 20% or more. According to the present invention, the correction of adenine (A) at position 3460 of mitochondrial ND1 DNA, adenine (A) at position 11778 of mitochondrial ND4 DNA, or cytosine (A) at position 14484 of mitochondrial ND6 DNA means that the corresponding base has changed as compared to the base sequence of the mitochondrial DNA that has not been contacted with the base editing composition according to the present invention. Whether the base has changed may be confirmed by DNA sequencing.

The present invention provides a method for preventing or treating Leber hereditary optic neuropathy (LHON), comprising administering to a patient in need of prevention or treatment of LHON an effective amount of a mitochondrial DNA base editor (or base editing composition; for example, a combination of fusion proteins or a combination of polynucleotides described under the section "Composition examples of Base Editor (or Dase Editing Composition) According to The Present Invention"), that is, a fusion protein (including a combination of one or more different fusion proteins) capable of correcting adenine (A) at position 3460 of mitochondrial ND1 DNA of a patient with LHON to guanine (G), or correcting adenine (A) at position 11778 of mitochondrial ND4 DNA to guanine (G), or correcting cytosine (A) at position 14484 of mitochondrial ND6 DNA to thymine (T), or a polynucleotide comprising a gene encoding the fusion protein (including a combination of different polynucleotides each encoding one or more different fusion proteins).

The term "effective amount" as used herein refers to an amount of a biologically active agent sufficient to induce a desired biological response. In some embodiments, the effective amount is the amount necessary to improve symptoms of the disease in an untreated patient. A therapeutic method of treating a disease and the effective amount of the active ingredient used in the treatment may vary depending on the method of administration and a subject's age, weight, and general health. In one embodiment, an effective amount is an amount of a base editor (e.g., a fusion protein, or polynucleotide, or a vector or lipid nanoparticle comprising the same) described herein sufficient to introduce a change in a gene of interest (e.g., mitochondrial DNA) in a cell (e.g., in vitro, in vivo or ex vivo). In one embodiment, the effective amount is the amount of the base editor (a fusion protein, or a polynucleotide, or a vector or lipid nanoparticle comprising the same) necessary to achieve a therapeutic effect (for example, to reduce or control symptoms or conditions of a patient with LHON). Such an therapeutic effect need not be sufficient to alter all mitochondrial DNA in all cells of the subject, tissue or organ, but may be sufficient to alter mitochondrial DNA in at least about 1%, 5%, 10%, 25%, 50%, 75%, or more of the cells present in the subject, tissue or organ, or may be sufficient to alter mitochondrial DNA in at least about 1%, 5%, 10%, 25%, 50%, 75%, or more of the total number of copies of mitochondrial DNA present in the corresponding cells. In one embodiment, the effective amount is sufficient to improve one or more symptoms of LHON.

The present invention provides a pharmaceutical composition for preventing or treating LHON comprising an effective amount of the base editing composition or the polynucleotide. The present invention provides a pharmaceutical composition for preventing or treating LHON, comprising a mitochondrial DNA base editor according to the present invention, i.e., a fusion protein (including a combination of one or more different fusion proteins) capable of editing adenine (A) at position 3460 of mitochondrial ND1 DNA of a patient with LHON to guanine (G), adenine (A) at position 11778 of mitochondrial ND4 DNA to guanine (G), or cytosine (A) at position 14484 of mitochondrial ND6 DNA to thymine (T), or a polynucleotide comprising a gene encoding the fusion protein (including a combination of different polynucleotides each encoding one or more different fusion proteins), and a pharmaceutically acceptable excipient, carrier or vehicle.

As used herein, the term "pharmaceutical composition" means a composition formulated for pharmaceutical use. In some embodiments, the pharmaceutical composition further comprises a pharmaceutically acceptable carrier. Those skilled in the pharmaceutical art are well aware of pharmaceutical carriers which may be generally used to formulate a base editing composition according to the present invention for pharmaceutical uses. In some embodiments, the pharmaceutical composition may comprise an additional agent (e.g., an agent for specific delivery, increasing half-life, or other therapeutic compounds). The term "pharmaceutically acceptable carrier" means a pharmaceutically acceptable substance, composition, or vehicle, such as a liquid or solid filler, diluent, excipient, manufacturing aid, or solvent encapsulating material, which carries or transports a compound from one site in the body (e.g., a site of delivery) to another site (e.g., an organ, tissue, or part of the body). A pharmaceutically acceptable carrier is "acceptable" in the sense that it is compatible with the other ingredients of the formulation and not deleterious to the tissues of the subject (e.g., in terms of physiological compatibility, sterility, physiological pH, etc.). The terms "excipient", "carrier", "pharmaceutically acceptable carrier", "vehicle", and the like may be used interchangeably.

The present invention provides a gene delivery vehicle comprising a polynucleotide encoding any one or more fusion proteins included in a base editing composition according to the present invention. The gene delivery vehicle may be a viral vector, preferably an adeno-associated viral vector. The gene delivery vehicle may also be in the form of a lipid nanoparticle or a polymeric nanoparticle. For example, a polynucleotide or combination of polynucleotides according to the present invention may form a complex with a lipid or a polymer.

The present invention provides a gene therapy agent for preventing or treating LHON, comprising the gene delivery vehicle. The pharmaceutical composition described above may be in the form of a gene therapy product comprising, as an active ingredient, a polynucleotide (including a combination of different polynucleotides each encoding one or more different fusion proteins) encoding a fusion protein (including a combination of one or more different fusion proteins) used as a mitochondrial DNA base editor according to the present invention.

According to the present invention, a polynucleotide comprising a gene encoding a fusion protein (including a combination of one or more different fusion proteins) used as a mitochondrial DNA base editor may be delivered to a patient, and as such delivery methods, a method using a virus as a vector, a non-viral method using a synthetic phospholipid, synthetic cationic polymer, or the like, an electroporation method in which a gene is introduced by temporarily stimulating the cell membrane electrically, and the like may be used. Among the above methods, when a virus is used as a vector, a virus with a low gene loading capacity (for example, an adeno-associated virus (AAV) with a size of approximately 4.7 kbp) has a limitation in use due to the size of a DNA editing fusion protein, but if a zinc finger protein is used as the DNA binding protein or a full-length deaminase is used as the deaminase, such a virus may be used as a vector. The vector may be an adeno-associated virus.

The present invention may relate to (1) to (35) below based on the contents described above, but is not limited thereto.
(1) A base editing composition capable of correcting a mitochondrial DNA mutation in a patient with Leber hereditary optic neuropathy (LHON), comprising one or more fusion proteins, wherein each of the one or more fusion proteins independently comprises DNA binding protein that specifically binds to mitochondrial DNA of a patient with LHON and further comprises at least one of adenine deaminase and cytosine deaminase, and wherein cytosine deaminase is present in a full-length form or in the form of two splits.
(2) In the base editing composition according to (1), wherein the composition is capable of editing adenine (A) at position 3460 of mitochondrial ND1 DNA to guanine (G), adenine (A) at position 11778 of mitochondrial ND4 DNA to guanine (G), or cytosine (A) at position 14484 of mitochondrial ND6 DNA to thymine (T) in a patient with LHON.
(3) In the base editing composition according to (1) or (2), wherein the cytosine deaminase is APOBEC (apolipoprotein B editing complex), AID (activation-induced deaminase), TadA (tRNA-specific adenosine deaminase) or DddAₜₒₓ, or a variant thereof.
(4) The base editing composition according to (1) or (2), wherein cytosine deaminase is DddAtox and is included in the form of a first split and a second split, and wherein one or more amino acids located on the interface between the first and second splits are substituted with other amino acids.
(5) The base editing composition according to (4), wherein the first split of DddAₜₒₓ comprises an amino acid sequence of SEQ ID NO: 5 or 139 or a variant thereof, wherein the variant has at least one amino acid selected from the group consisting of positions 87, 88, 91, 92, 95, 100, 101, 102, and 103 of the amino acid sequence of SEQ ID NO: 5 substituted with another amino acid, and the second split of DddAₜₒₓ comprises an amino acid sequence of SEQ ID NO: 6 or 140 or a variant thereof, wherein the variant has at least one amino acid selected from the group consisting of positions 13, 14, 15, and 16 of the amino acid sequence of SEQ ID NO: 6 substituted with another amino acid.
(6) The base editing composition according to (1) or (2), wherein the adenine deaminase is APOBEC, AID or TadA, or a variant thereof.
(7) The base editing composition according to (6),wherein the adenine deaminase comprises the amino acid sequence of SEQ ID NO: 1 or a conservative amino acid substitution thereof.
(8) The base editing composition according to any one of (1) to (7), wherein the DNA binding protein is selected from the group consisting of a zinc finger protein, a TALE protein, and a CRISPR-associated nuclease.
(9) The base editing composition according to any one of (1) to (8), wherein each of the one or more fusion proteins independently comprises UGI (uracil glycosylase inhibitor).
(10) The base editing composition according to any one of (1) to (9), wherein each of the one or more fusion proteins independently comprises a nuclear export signal (NES).
(11) The base editing composition according to any one of claims (1) to (10), wherein each of the one or more fusion proteins independently comprises a mitochondrial targeting sequence (MTS).
(12) The base editing composition according to any one of (1) to (8), wherein one DNA binding protein binds to a nucleotide sequence of mitochondrial ND1 DNA: 
5'-
CAAACTCAAACTACGAACGCACTCACAGTCACATCATAATCCTCTCTCAAGGACTT
CAAAC-3'
 or a portion thereof.
(13) The base editing composition according to any one of (1) to (8), wherein one DNA binding protein binds to a nucleotide sequence of mitochondrial ND1 DNA: 
5'-
CAAACTCAAACTACGAACGCACTCACAGTCACATCATAATCCTCTCTCAAGGACTT
CAAAC-3'
 or a portion thereof.
(14) The base editing composition according to any one of (1) to (8), wherein one DNA binding protein binds to a nucleotide sequence of mitochondrial ND1 DNA: 
5'-
TCGCTGTAGTATATCCAAAGACAACCACCATTCCCCCTAAATAAATTAAAAAAACT-
3'
 or a portion thereof.
(15) The base editing composition according to any one of (1) to (8), wherein one DNA binding protein comprises an amino acid sequence selected from the group consisting of SEQ ID NOs: 7 to 65, or a conservative amino acid substitution thereof.
(16) The base editing composition according to (1), wherein the composition comprises two fusion proteins and is capable of editing adenine (A) at position 3460 of mitochondrial ND1 DNA to guanine (G) in a patient with LHON, wherein each of the two fusion proteins comprises DddAtox split and TALE protein that specifically binds to mitochondrial ND1 DNA, and one of the two fusion proteins further comprises TadA8e.
(17) The base editing composition according to (1), wherein the composition comprises two fusion proteins and is capable of editing adenine (A) at position 11778 of mitochondrial ND4 DNA to guanine (G) in a patient with LHON, wherein each of the two fusion proteins comprises DddAtox split and TALE protein or zinc finger protein that specifically binds to mitochondrial ND4 DNA, and one of the two fusion proteins further comprises TadA8e.
(18) The base editing composition according to (1), wherein two fusion proteins, wherein the composition comprises two fusion proteins and is capable of editing cytosine (A) at position 14484 of mitochondrial ND6 DNA to thymine (T) in a patient with LHON, wherein each of the two fusion proteins comprises DddAtox split and TALE protein that specifically binds to mitochondrial ND6.
(19) The base editing composition according to (16), wherein one fusion protein comprises TALE protein comprising an amino acid sequence selected from the group consisting of SEQ ID NOs: 10 to 17 or conservative amino acid substitution thereof, and the other fusion protein comprises TALE protein comprising an amino acid sequence selected from the group consisting of SEQ ID NOs: 18 to 31 or a conservative amino acid substitution thereof.
(20) The base editing composition according to (17), wherein one fusion protein comprises TALE protein comprising an amino acid sequence selected from the group consisting of SEQ ID NOs: 32 to 42 or zinc finger protein comprising an amino acid sequence selected from the group consisting of SEQ ID NOs: 7 to 9 or conservative amino acid substitution thereof, and the other fusion protein comprises TALE protein comprising an amino acid sequence selected from the group consisting of SEQ ID NOs: 43 to 53 or zinc finger protein comprising an amino acid sequence selected from the group consisting of SEQ ID NOs: 7 to 9 or a conservative amino acid substitution thereof.
(21) The base editing composition according to (18), wherein one fusion protein comprises a TALE protein comprising an amino acid sequence selected from the group consisting of SEQ ID NOs: 54 to 57 or a conservative amino acid substitution thereof, and the other fusion protein comprises a TALE protein comprising an amino acid sequence selected from the group consisting of SEQ ID NOs: 58 to 65 or a conservative amino acid substitution thereof.
(22) The base editing composition according to (16) or (19), comprising a combination of a fusion protein comprising an amino acid sequence selected from the group consisting of SEQ ID NOs: 78 to 85 and a fusion protein comprising an amino acid sequence selected from the group consisting of SEQ ID NOs: 86 to 99.
(23) The base editing composition according to (17) or (20), comprising a combination of a fusion protein comprising an amino acid sequence selected from the group consisting of SEQ ID NOs: 100 to 114 and a fusion protein comprising an amino acid sequence selected from the group consisting of SEQ ID NOs: 115 to 125.
(24) The base editing composition according to (18) or (21), comprising a combination of a fusion protein comprising an amino acid sequence selected from the group consisting of SEQ ID NOs: 66 to 69 and a fusion protein comprising an amino acid sequence selected from the group consisting of SEQ ID NOs: 70 to 77.
(25) A polynucleotide encoding any one of the fusion proteins included in the base editing composition according to any one of (1) to (24), or a combination of two or more of said polynucleotides.
(26) The combination of polynucleotides according to (25), comprising a polynucleotide encoding an amino acid sequence selected from the group consisting of SEQ ID NOs: 78 to 85 and a polynucleotide encoding an amino acid sequence selected from the group consisting of SEQ ID NOs: 86 to 99.
(27) The combination of polynucleotides according to (25), comprising a polynucleotide encoding an amino acid sequence selected from the group consisting of SEQ ID NOs: 100 to 114 and a polynucleotide encoding an amino acid sequence selected from the group consisting of SEQ ID NOs: 115 to 125.
(28) The combination of polynucleotides according to (25), comprising a polynucleotide encoding an amino acid sequence selected from the group consisting of SEQ ID NOs: 66 to 69 and a polynucleotide encoding an amino acid sequence selected from the group consisting of SEQ ID NOs: 70 to 77.
(29) A method of correcting a mitochondrial DNA mutation in a patient with LHON, the method comprising contacting mitochondrial DNA of the patient with LHON with a base editing composition according to any one of (1) to (24), wherein the correction involves editing of guanine (G) at position 3460 of mitochondrial ND1 DNA to adenine (A), guanine (G) at position 11778 of mitochondrial ND4 DNA to adenine (A), or cytosine (A) at position 14484 of mitochondrial ND6 DNA to thymine (T).
(30) A method of preventing or treating LHON, comprising administering to a patient in need of prevention or treatment of LHON a base editing composition according to any one of (1) to (24) or a composition comprising a polynucleotide or a combination of polynucleotides according to any one of (25) to (28).
(31) A pharmaceutical composition for preventing or treating LHON, comprising a base editing composition according to any one of claims (1) to (24) or a polynucleotide or combination of polynucleotides according to any one of claims (25) to (28).
(32) gene delivery vehicle comprising a polynucleotide or a combination of polynucleotides according to any one of (25) to (28).
(33) The gene delivery vehicle according to (32), wherein the gene delivery vehicle is an adeno-associated virus vector.
(34) The gene delivery vehicle according to (32), wherein the gene delivery vehicle is a lipid nanoparticle or a polymeric nanoparticle.
(35) A gene therapy agent for preventing or treating LHON, comprising a gene delivery vehicle according to (32) to (34).

Hereinafter, embodiments of the present invention will be described. However, the following examples are provided only to illustrate the present invention, and should not be construed as limiting the scope of the present invention.

### Example 1: Amplification of template DNA having the m.T14484C mutation

A double-stranded DNA sequence mimicking the mitochondrial genome of a patient with the m.T14484C mutation was synthesized as a gBlock DNA fragment from IDT (Integrated DNA Technologies). The sequence of the obtained template DNA is as follows.

Template DNA was amplified using the forward primer (GACTGGTTCCAATTGACAACG) and reverse primer (GCAAATGGCATTCTGACATCC), and then purified using a PCR purification kit (Geneall). It was freshly diluted in distilled water to a concentration of 10 ng/µL just before use in the experiment.

### Example 2: Synthesis of fusion proteins to correct the m.T14484C mutation

After adjusting the concentration of the template DNA obtained in Example 1 to 10 ng/µL, a reaction mixture containing 10 ng of template DNA, 0.5 µg of a plasmid containing DNA encoding the first fusion protein, 0.5 µg of a plasmid containing DNA encoding the second fusion protein, and 20 µL of an in vitro coupled transcription/translation (IVTT) kit mixture (including distilled water up to 25 µL) was mixed in a tube, and then reacted at 30°C for 6 hours and then at 37°C for 16 hours.

The first fusion protein and the second fusion protein were linked in the order of [MTS]-[tag]-[TALE protein]-[linker]-[DddAₜₒₓ split]-[linker]-[UGI]. The tag is 3X HA(SEQ ID NO: 136) or 3X FLAG (SEQ ID NO: 137). The proteins used are located between the CMV promoter and the T7 promoter and terminator sequence.

### Example 3: Sequencing and Base Editing Efficiency Measurement to Confirm Correction of m.T14484C Mutation

The reaction product obtained in Example 2 was as a template without purification, and the sequence was analyzed using a targeted deep sequencing technique. The efficiency of the base editors developed for m.C14484T correction was screened, and the DNA binding sites and base editing efficiencies of 15 base editors with high efficiency are shown in FIG. 1.

The amino acid sequences of the first fusion protein (including the left TALE) and the second fusion protein (including the right TALE) used in the 15 base editors that were confirmed to have high base editing efficiency are as follows.

### First Fusion Protein

SEQ ID NO: 66 
SEQ ID NO: 66

MALSRAVCGTSRQLAPVLGYLGSRQKHSLPDYPYDVPDYAGYPYDVP

DYAGYPYDVPDYAGIRIQDLRTLGYSQQQQEKIKPKVRSTVAQHHEALVGHGF

THAHIVALSQHPAALGTVAVKYQDMIAALPEATHEAIVGVGKQWSGARALEA

LLTVAGELRGPPLQLDTGQLLKIAKRGGVTAVEAVHAWRNALTGAPLNLTPAQ

VVAIASNIGGKQALETVQRLLPVLCQAHGLTPEQVVAIASNNGGKQALETVQR

LLPVLCQAHGLTPDQVVAIASNGGGKQALETVQRLLPVLCQDHGLTPAQVVAI

ASNIGGKQALETVQRLLPVLCQDHGLTPAQVVAIASNGGGKQALETVQRLLPV

LCQDHGLTPDQVVAIASNIGGKQALETVQRLLPVLCQDHGLTPAQVVAIASNG

GGKQALETVQRLLPVLCQDHGLTPAQVVAIASHDGGKQALETVQRLLPVLCQ

AHGLTPEQVVAIASHDGGKQALETVQRLLPVLCQAHGLTPDQVVAIASNIGGK

QALETVQRLLPVLCQAHGLTPAQVVAIASNIGGKQALETVQRLLPVLCQDHGL

TPAQVVAIASNIGGKQALETVQRLLPVLCQAHGLTPAQVVAIASNNGGKQALE

TVQRLLPVLCQAHGLTPAQVVAIASNIGGKQALETVQRLLPVLCQAHGLTPAQ

VVAIASHDGGKQALETVQRLLPVLCQAHGLTPEQVVAIASNGGGKQALESIVA

QLSRPDPALAALTNDHLVALACLGGRPALDAVKKGLGGSGSGSYALGPYQISA

PQLPAYNGQTVGTFYYVNDAGGLESKVFISGGPTPYPNYVSAGHVEGQSALF
 
MRDNGISEGLVFHNNPKGTCGFCVNMIETLLPENAAMTVVPPEGSGGSTNLSD
IIEKETGKQLVIQESILMLPEEVEEVIGNKPESDILVHTAYDESTDENVMLLTSDA
PEYKPWALVIQDSNGENKIKML
 (The underlined portion corresponds to the TALE protein including the N-terminal domain and C-terminal domain (including the half domain))
SEQ ID NO: 67 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKQWSGARALEA
LLTVAGELRGPPLQLDTGQLLKIAKRGGVTAVEAVHAWRNALTGAPLNLTPAQ
VVAIASNIGGKQALETVQRLLPVLCQAHGLTPEQVVAIASNNGGKQALETVQR
LLPVLCQAHGLTPDQVVAIASNGGGKQALETVQRLLPVLCQDHGLTPAQVVAI
ASNIGGKQALETVQRLLPVLCQDHGLTPAQVVAIASNGGGKQALETVQRLLPV
LCQDHGLTPDQVVAIASNIGGKQALETVQRLLPVLCQDHGLTPAQVVAIASNG
GGKQALETVQRLLPVLCQDHGLTPAQVVAIASHDGGKQALETVQRLLPVLCQ
AHGLTPEQVVAIASHDGGKQALETVQRLLPVLCQAHGLTPDQVVAIASNIGGK
QALETVQRLLPVLCQAHGLTPAQVVAIASNIGGKQALETVQRLLPVLCQDHGL
TPAQVVAIASNIGGKQALETVQRLLPVLCQAHGLTPDQVVAIASNNGGKQALE
TVQRLLPVLCQAHGLTPDQVVAIASNIGGKQALETVQRLLPVLCQAHGLTPEQ
VVAIASNGGGKQALESIVAQLSRPDPALAALTNDHLVALACLGGRPALDAVKK
GLGGSGSGSYALGPYQISAPQLPAYNGQTVGTFYYVNDAGGLESKVFISGGPTP
YPNYVSAGHVEGQSALFMRDNGISEGLVFHNNPKGTCGFCVNMIETLLPENA
AMTVVPPEGSGGSTNLSDIIEKETGKQLVIQESILMLPEEVEEVIGNKPESDILVH
TAYDESTDENVMLLTSDAPEYKPWALVIQDSNGENKIKML
 (The underlined portion corresponds to the TALE protein including the N-terminal domain and C-terminal domain (including the half domain))
SEQ ID NO: 68. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPAQVVAIASHD
GGKQALETVQRLLPVLCQAHGLTPDQVVAIASNNGGKQALETVQRLLPVLCQ
DHGLTPDQVVAIASHDGGKQALETVQRLLPVLCQDHGLTPAQVVAIASNGGG
KQALETVQRLLPVLCQAHGLTPDQVVAIASNNGGKQALETVQRLLPVLCQAH
GLTPAQVVAIASNGGGKQALETVQRLLPVLCQDHGLTPAQVVAIASNIGGKQA
LETVQRLLPVLCQAHGLTPEQVVAIASNNGGKQALETVQRLLPVLCQAHGLTP
DQVVAIASNGGGKQALETVQRLLPVLCQAHGLTPAQVVAIASNIGGKQALETV
QRLLPVLCQDHGLTPAQVVAIASNGGGKQALETVQRLLPVLCQDHGLTPDQV
VAIASNIGGKQALETVQRLLPVLCQDHGLTPAQVVAIASNGGGKQALETVQRL
LPVLCQDHGLTPEQVVAIASHDGGKQALETVQRLLPVLCQAHGLTPAQVVAIA
SHDGGKQALETVQRLLPVLCQDHGLTPDQVVAIASNIGGKQALETVQRLLPVL
CQAHGLTPEQVVAIASNGGGKQALESIVAQLSRPDPALAALTNDHLVALACLG
GRPALDAVKKGLGGSGSAIPVKRGATGETKVFIGNSNSPKSPTKGGCSGGSTNL
SDIIEKETGKQLVIQESILMLPEEVEEVIGNKPESDILVHTAYDESTDENVMLLTS
DAPEYKPWALVIQDSNGENKIKML
 (The underlined portion corresponds to the TALE protein including the N-terminal domain and C-terminal domain (including the half domain))
SEQ ID NO: 69. 
MASVLTPLLLRGLTGSARRLPVPRAKIHSLDYKDHDGDYKDHDIDYK
DDDDKGIRIQDLRTLGYSQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPAQVVAIASHD
 
GGKQALETVQRLLPVLCQAHGLTPDQVVAIASNNGGKQALETVQRLLPVLCQ
DHGLTPDQVVAIASHDGGKQALETVQRLLPVLCQDHGLTPAQVVAIASNGGG
KQALETVQRLLPVLCQAHGLTPDQVVAIASNNGGKQALETVQRLLPVLCQAH
GLTPAQVVAIASNGGGKQALETVQRLLPVLCQDHGLTPAQVVAIASNIGGKQA
LETVQRLLPVLCQAHGLTPEQVVAIASNNGGKQALETVQRLLPVLCQAHGLTP
DQVVAIASNGGGKQALETVQRLLPVLCQAHGLTPAQVVAIASNIGGKQALETV
QRLLPVLCQDHGLTPAQVVAIASNGGGKQALETVQRLLPVLCQDHGLTPDQV
VAIASNIGGKQALETVQRLLPVLCQDHGLTPAQVVAIASNGGGKQALETVQRL
LPVLCQDHGLTPEQVVAIASHDGGKQALETVQRLLPVLCQAHGLTPAQVVAIA
SHDGGKQALETVQRLLPVLCQAHGLTPEQVVAIASNGGGKQALESIVAQLSRP
DPALAALTNDHLVALACLGGRPALDAVKKGLGGSGSAIPVKRGATGETKVFIG
NSNSPKSPTKGGCSGGSTNLSDIIEKETGKQLVIQESILMLPEEVEEVIGNKPESD
ILVHTAYDESTDENVMLLTSDAPEYKPWALVIQDSNGENKIKML
 (The underlined portion corresponds to the TALE protein including the N-terminal domain and C-terminal domain (including the half domain))

### Second Fusion Protein

SEQ ID NO: 70. 
MASVLTPLLLRGLTGSARRLPVPRAKIHSLDYKDHDGDYKDHDIDYK
DDDDKGIRIQDLRTLGYSQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASNI
GGKQALETVQRLLPVLCQAHGLTPDQVVAIASNIGGKQALETVQRLLPVLCQD
HGLTPDQVVAIASNGGGKQALETVQRLLPVLCQAHGLTPEQVVAIASNGGGK
QALETVQRLLPVLCQAHGLTPDQVVAIASNGGGKQALETVQRLLPVLCQDHG
LTPDQVVAIASNIGGKQALETVQRLLPVLCQDHGLTPDQVVAIASNGGGKQAL
 
ETVQRLLPVLCQAHGLTPAQVVAIASNGGGKQALETVQRLLPVLCQAHGLTPE
QVVAIASNGGGKQALETVQRLLPVLCQAHGLTPAQVVAIASNIGGKQALETVQ
RLLPVLCQAHGLTPAQVVAIASNNGGKQALETVQRLLPVLCQDHGLTPAQVVA
IASNNGGKQALETVQRLLPVLCQAHGLTPAQVVAIASNNGGKQALETVQRLLP
VLCQAHGLTPAQVVAIASNNGGKQALETVQRLLPVLCQAHGLTPDQVVAIASN
NGGKQALETVQRLLPVLCQAHGLTPEQVVAIASNGGGKQALESIVAQLSRPDP
ALAALTNDHLVALACLGGRPALDAVKKGLGGSGSAIPVKRGATGETKVFIGNS
NSPKSPTKGGCSGGSTNLSDIIEKETGKQLVIQESILMLPEEVEEVIGNKPESDIL
VHTAYDESTDENVMLLTSDAPEYKPWALVIQDSNGENKIKML
 (The underlined portion corresponds to the TALE protein including the N-terminal domain and C-terminal domain (including the half domain))
SEQ ID NO: 71. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPEQVVAIASNG
GGKQALETVQRLLPVLCQAHGLTPAQVVAIASNIGGKQALETVQRLLPVLCQD
HGLTPAQVVAIASNIGGKQALETVQRLLPVLCQAHGLTPDQVVAIASNGGGKQ
ALETVQRLLPVLCQAHGLTPAQVVAIASNGGGKQALETVQRLLPVLCQAHGLT
PEQVVAIASNGGGKQALETVQRLLPVLCQAHGLTPAQVVAIASNIGGKQALET
VQRLLPVLCQAHGLTPDQVVAIASNGGGKQALETVQRLLPVLCQAHGLTPDQ
VVAIASNGGGKQALETVQRLLPVLCQAHGLTPAQVVAIASNGGGKQALETVQ
RLLPVLCQDHGLTPAQVVAIASNIGGKQALETVQRLLPVLCQAHGLTPDQVVAI
ASNNGGKQALETVQRLLPVLCQAHGLTPAQVVAIASNNGGKQALETVQRLLP
VLCQAHGLTPAQVVAIASNNGGKQALETVQRLLPVLCQAHGLTPAQVVAIASN
 
NGGKQALETVQRLLPVLCQAHGLTPAQVVAIASNNGGKQALETVQRLLPVLC
QAHGLTPEQVVAIASNGGGKQALESIVAQLSRPDPALAALTNDHLVALACLGG
RPALDAVKKGLGGSGSAIPVKRGATGETKVFIGNSNSPKSPTKGGCSGGSTNLS
DIIEKETGKQLVIQESILMLPEEVEEVIGNKPESDILVHTAYDESTDENVMLLTSD
APEYKPWALVIQDSNGENKIKML
 (The underlined portion corresponds to the TALE protein including the N-terminal domain and C-terminal domain (including the half domain))
SEQ ID NO: 72. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPEQVVAIASNG
GGKQALETVQRLLPVLCQAHGLTPDQVVAIASNGGGKQALETVQRLLPVLCQ
DHGLTPDQVVAIASNIGGKQALETVQRLLPVLCQDHGLTPAQVVAIASNIGGKQ
ALETVQRLLPVLCQAHGLTPDQVVAIASNGGGKQALETVQRLLPVLCQAHGL
TPDQVVAIASNGGGKQALETVQRLLPVLCQAHGLTPDQVVAIASNGGGKQAL
ETVQRLLPVLCQAHGLTPDQVVAIASNIGGKQALETVQRLLPVLCQDHGLTPD
QVVAIASNGGGKQALETVQRLLPVLCQAHGLTPEQVVAIASNGGGKQALETV
QRLLPVLCQAHGLTPDQVVAIASNGGGKQALETVQRLLPVLCQDHGLTPDQV
VAIASNIGGKQALETVQRLLPVLCQDHGLTPAQVVAIASNNGGKQALETVQRL
LPVLCQAHGLTPDQVVAIASNNGGKQALETVQRLLPVLCQDHGLTPEQVVAIA
SNNGGKQALETVQRLLPVLCQAHGLTPAQVVAIASNNGGKQALETVQRLLPV
LCQAHGLTPAQVVAIASNNGGKQALETVQRLLPVLCQAHGLTPEQVVAIASNG
GGKQALESIVAQLSRPDPALAALTNDHLVALACLGGRPALDAVKKGLGGSGSA
IPVKRGATGETKVFIGNSNSPKSPTKGGCSGGSTNLSDIIEKETGKQLVIQESILM
LPEEVEEVIGNKPESDILVHTAYDESTDENVMLLTSDAPEYKPWALVIQDSNGE
 NKIKML (The underlined portion corresponds to the TALE protein including the N-terminal domain and C-terminal domain (including the half domain))
SEQ ID NO: 73. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPEQVVAIASNG
GGKQALETVQRLLPVLCQAHGLTPDQVVAIASNGGGKQALETVQRLLPVLCQ
DHGLTPDQVVAIASNIGGKQALETVQRLLPVLCQDHGLTPAQVVAIASNIGGKQ
ALETVQRLLPVLCQAHGLTPDQVVAIASNGGGKQALETVQRLLPVLCQAHGL
TPDQVVAIASNGGGKQALETVQRLLPVLCQAHGLTPDQVVAIASNGGGKQAL
ETVQRLLPVLCQAHGLTPDQVVAIASNIGGKQALETVQRLLPVLCQDHGLTPD
QVVAIASNGGGKQALETVQRLLPVLCQAHGLTPEQVVAIASNGGGKQALETV
QRLLPVLCQAHGLTPDQVVAIASNGGGKQALETVQRLLPVLCQDHGLTPDQV
VAIASNIGGKQALETVQRLLPVLCQDHGLTPAQVVAIASNNGGKQALETVQRL
LPVLCQAHGLTPAQVVAIASNNGGKQALETVQRLLPVLCQAHGLTPAQVVAIA
SNNGGKQALETVQRLLPVLCQAHGLTPAQVVAIASNNGGKQALETVQRLLPV
LCQAHGLTPEQVVAIASNGGGKQALESIVAQLSRPDPALAALTNDHLVALACLG
GRPALDAVKKGLGGSGSAIPVKRGATGETKVFIGNSNSPKSPTKGGCSGGSTNL
SDIIEKETGKQLVIQESILMLPEEVEEVIGNKPESDILVHTAYDESTDENVMLLTS
DAPEYKPWALVIQDSNGENKIKML
 (The underlined portion corresponds to the TALE protein including the N-terminal domain and C-terminal domain (including the half domain))
SEQ ID NO: 74. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
 
THAHIVALSQHPAALGTVAVKYQDMIAALPEATHEAIVGVGKQWSGARALEA
LLTVAGELRGPPLQLDTGQLLKIAKRGGVTAVEAVHAWRNALTGAPLNLTPDQ
VVAIASNGGGKQALETVQRLLPVLCQAHGLTPAQVVAIASNGGGKQALETVQ
RLLPVLCQAHGLTPEQVVAIASNGGGKQALETVQRLLPVLCQAHGLTPEQVVA
IASNGGGKQALETVQRLLPVLCQAHGLTPAQVVAIASNIGGKQALETVQRLLP
VLCQDHGLTPAQVVAIASNIGGKQALETVQRLLPVLCQAHGLTPDQVVAIASN
GGGKQALETVQRLLPVLCQAHGLTPAQVVAIASNGGGKQALETVQRLLPVLC
QAHGLTPEQVVAIASNGGGKQALETVQRLLPVLCQAHGLTPAQVVAIASNIGG
KQALETVQRLLPVLCQAHGLTPDQVVAIASNGGGKQALETVQRLLPVLCQAH
GLTPDQVVAIASNGGGKQALETVQRLLPVLCQAHGLTPAQVVAIASNGGGKQ
ALETVQRLLPVLCQDHGLTPAQVVAIASNIGGKQALETVQRLLPVLCQAHGLT
PDQVVAIASNNGGKQALETVQRLLPVLCQAHGLTPAQVVAIASNNGGKQALE
TVQRLLPVLCQAHGLTPDQVVAIASNNGGKQALETVQRLLPVLCQDHGLTPEQ
VVAIASNNGGKQALETVQRLLPVLCQAHGLTPEQVVAIASNGGGKQALESIVA
QLSRPDPALAALTNDHLVALACLGGRPALDAVKKGLGGSGSGSYALGPYQISA
PQLPAYNGQTVGTFYYVNDAGGLESKVFISGGPTPYPNYVSAGHVEGQSALF
MRDNGISEGLVFHNNPKGTCGFCVNMIETLLPENAAMTVVPPEGSGGSTNLSD
IIEKETGKQLVIQESILMLPEEVEEVIGNKPESDILVHTAYDESTDENVMLLTSDA
PEYKPWALVIQDSNGENKIKML
 (The underlined portion corresponds to the TALE protein including the N-terminal domain and C-terminal domain (including the half domain))
SEQ ID NO: 75. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKQWSGARALEA
LLTVAGELRGPPLQLDTGQLLKIAKRGGVTAVEAVHAWRNALTGAPLNLTPDQ
 
VVAIASNGGGKQALETVQRLLPVLCQAHGLTPAQVVAIASNGGGKQALETVQ
RLLPVLCQAHGLTPEQVVAIASNGGGKQALETVQRLLPVLCQAHGLTPEQVVA
IASNGGGKQALETVQRLLPVLCQAHGLTPAQVVAIASNIGGKQALETVQRLLP
VLCQDHGLTPAQVVAIASNIGGKQALETVQRLLPVLCQAHGLTPDQVVAIASN
GGGKQALETVQRLLPVLCQAHGLTPAQVVAIASNGGGKQALETVQRLLPVLC
QAHGLTPEQVVAIASNGGGKQALETVQRLLPVLCQAHGLTPAQVVAIASNIGG
KQALETVQRLLPVLCQAHGLTPDQVVAIASNGGGKQALETVQRLLPVLCQAH
GLTPDQVVAIASNGGGKQALETVQRLLPVLCQAHGLTPAQVVAIASNGGGKQ
ALETVQRLLPVLCQDHGLTPAQVVAIASNIGGKQALETVQRLLPVLCQAHGLT
PDQVVAIASNNGGKQALETVQRLLPVLCQAHGLTPAQVVAIASNNGGKQALE
TVQRLLPVLCQAHGLTPAQVVAIASNNGGKQALETVQRLLPVLCQAHGLTPEQ
VVAIASNGGGKQALESIVAQLSRPDPALAALTNDHLVALACLGGRPALDAVKK
GLGGSGSGSYALGPYQISAPQLPAYNGQTVGTFYYVNDAGGLESKVFISGGPTP
YPNYVSAGHVEGQSALFMRDNGISEGLVFHNNPKGTCGFCVNMIETLLPENA
AMTVVPPEGSGGSTNLSDIIEKETGKQLVIQESILMLPEEVEEVIGNKPESDILVH
TAYDESTDENVMLLTSDAPEYKPWALVIQDSNGENKIKML
 (The underlined portion corresponds to the TALE protein including the N-terminal domain and C-terminal domain (including the half domain))
SEQ ID NO:76. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKQWSGARALEA
LLTVAGELRGPPLQLDTGQLLKIAKRGGVTAVEAVHAWRNALTGAPLNLTPDQ
VVAIASNGGGKQALETVQRLLPVLCQAHGLTPAQVVAIASNGGGKQALETVQ
RLLPVLCQAHGLTPEQVVAIASNGGGKQALETVQRLLPVLCQAHGLTPEQVVA
 
IASNGGGKQALETVQRLLPVLCQAHGLTPAQVVAIASNIGGKQALETVQRLLP
VLCQDHGLTPAQVVAIASNIGGKQALETVQRLLPVLCQAHGLTPDQVVAIASN
GGGKQALETVQRLLPVLCQAHGLTPAQVVAIASNGGGKQALETVQRLLPVLC
QAHGLTPEQVVAIASNGGGKQALETVQRLLPVLCQAHGLTPAQVVAIASNIGG
KQALETVQRLLPVLCQAHGLTPDQVVAIASNGGGKQALETVQRLLPVLCQAH
GLTPDQVVAIASNGGGKQALETVQRLLPVLCQAHGLTPAQVVAIASNGGGKQ
ALETVQRLLPVLCQDHGLTPAQVVAIASNIGGKQALETVQRLLPVLCQDHGLT
PAQVVAIASNNGGKQALETVQRLLPVLCQAHGLTPAQVVAIASNNGGKQALET
VQRLLPVLCQAHGLTPEQVVAIASNGGGKQALESIVAQLSRPDPALAALTNDH
LVALACLGGRPALDAVKKGLGGSGSGSYALGPYQISAPQLPAYNGQTVGTFYY
VNDAGGLESKVFISGGPTPYPNYVSAGHVEGQSALFMRDNGISEGLVFHNNPK
GTCGFCVNMIETLLPENAAMTVVPPEGSGGSTNLSDIIEKETGKQLVIQESILML
PEEVEEVIGNKPESDILVHTAYDESTDENVMLLTSDAPEYKPWALVIQDSNGEN
KIKML
 (The underlined portion corresponds to the TALE protein including the N-terminal domain and C-terminal domain (including the half domain))
SEQ ID NO: 77. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKQWSGARALEA
LLTVAGELRGPPLQLDTGQLLKIAKRGGVTAVEAVHAWRNALTGAPLNLTPDQ
VVAIASNGGGKQALETVQRLLPVLCQAHGLTPAQVVAIASNGGGKQALETVQ
RLLPVLCQAHGLTPEQVVAIASNGGGKQALETVQRLLPVLCQAHGLTPEQVVA
IASNGGGKQALETVQRLLPVLCQAHGLTPAQVVAIASNIGGKQALETVQRLLP
VLCQDHGLTPAQVVAIASNIGGKQALETVQRLLPVLCQAHGLTPDQVVAIASN
GGGKQALETVQRLLPVLCQAHGLTPAQVVAIASNGGGKQALETVQRLLPVLC
 
QAHGLTPEQVVAIASNGGGKQALETVQRLLPVLCQAHGLTPAQVVAIASNIGG
KQALETVQRLLPVLCQAHGLTPDQVVAIASNGGGKQALETVQRLLPVLCQAH
GLTPDQVVAIASNGGGKQALETVQRLLPVLCQAHGLTPAQVVAIASNGGGKQ
ALETVQRLLPVLCQDHGLTPAQVVAIASNIGGKQALETVQRLLPVLCQDHGLT
PDQVVAIASNNGGKQALETVQRLLPVLCQAHGLTPEQVVAIASNGGGKQALES
IVAQLSRPDPALAALTNDHLVALACLGGRPALDAVKKGLGGSGSGSYALGPYQI
SAPQLPAYNGQTVGTFYYVNDAGGLESKVFISGGPTPYPNYVSAGHVEGQSAL
FMRDNGISEGLVFHNNPKGTCGFCVNMIETLLPENAAMTVVPPEGSGGSTNLS
DIIEKETGKQLVIQESILMLPEEVEEVIGNKPESDILVHTAYDESTDENVMLLTSD
APEYKPWALVIQDSNGENKIKML
 (The underlined portion corresponds to the TALE protein including the N-terminal domain and C-terminal domain (including the half domain))

The combinations of the first fusion protein and the second fusion protein used in the base editors shown in FIG. 1 are as follows.

| Base Editor ID | First Fusion Protein | Second Fusion Protein |
|---|---|---|
| 22-4-1 | SEQ ID NO: 66 | SEQ ID NO: 70 |
| 22-5-1 | SEQ ID NO: 67 | SEQ ID NO: 70 |
| 23-4-1 | SEQ ID NO: 66 | SEQ ID NO: 71 |
| 23-5-1 | SEQ ID NO: 67 | SEQ ID NO: 71 |
| 24-4-1 | SEQ ID NO: 66 | SEQ ID NO: 72 |
| 24-4-2 | SEQ ID NO: 66 | SEQ ID NO: 73 |
| 24-5-1 | SEQ ID NO: 67 | SEQ ID NO: 72 |
| 24-5-2 | SEQ ID NO: 67 | SEQ ID NO: 73 |
| 40-3-1 | SEQ ID NO: 68 | SEQ ID NO: 74 |
| 40-3-2 | SEQ ID NO: 68 | SEQ ID NO: 75 |
| 40-3-3 | SEQ ID NO: 68 | SEQ ID NO: 76 |
| 40-3-4 | SEQ ID NO: 68 | SEQ ID NO: 77 |
| 40-4-1 | SEQ ID NO: 69 | SEQ ID NO: 74 |
| 40-4-2 | SEQ ID NO: 69 | SEQ ID NO: 75 |
| 40-4-3 | SEQ ID NO: 69 | SEQ ID NO: 76 |

HEYWMRHALTLAKRARDEREVPVGAVLVLNNRVIGEGWNRAIGLHDPTAH
AEIMALRQGGLVMQNYRLIDATLYVTFEPCVMCAGAMIHSRIGRVVFGVRNSKRGAA
GSLMNVLNYPGMNHRVEITEGILADECAALLCDFYRMPRQVFNAQKKAQSSIN

(The underlined portion corresponds to the TALE protein including the N-terminal domain and C-terminal domain (including the half domain))
SEQ ID NO: 75. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPEQVVAIASNG
GGKQALETVQRLLPVLCQAHGLTPDQVVAIASNGGGKQALETVQRLLPVLCQ
DHGLTPDQVVAIASNIGGKQALETVQRLLPVLCQDHGLTPAQVVAIASNNGGK
QALETVQRLLPVLCQAHGLTPDQVVAIASNNGGKQALETVQRLLPVLCQDHG
LTPEQVVAIASNNGGKQALETVQRLLPVLCQAHGLTPDQVVAIASNNGGKQAL
ETVQRLLPVLCQAHGLTPAQVVAIASHDGGKQALETVQRLLPVLCQDHGLTPD
QVVAIASNGGGKQALETVQRLLPVLCQAHGLTPAQVVAIASHDGGKQALETV
QRLLPVLCQAHGLTPDQVVAIASNGGGKQALETVQRLLPVLCQAHGLTPAQV
VAIASNGGGKQALETVQRLLPVLCQDHGLTPEQVVAIASNGGGKQALETVQRL
LPVLCQAHGLTPAQVVAIASNNGGKQALETVQRLLPVLCQDHGLTPDQVVAIA
SNNGGKQALETVQRLLPVLCQAHGLTPDQVVAIASNGGGKQALETVQRLLPV
 
LCQAHGLTPEQVVAIASNGGGKQALESIVAQLSRPDPALAALTNDHLVALACLG
GRPALDAVKKGLLVGSAIPVKRGATGETKVFTGNSNSPKSPTKGGCSGSETPGT
SESATPESSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGVRNSKRGAAGSLMNVLNYPGMNHRVEITEGILADECAALLCDFYR
MPRQVFNAQKKAQSSIN
 (The underlined portion corresponds to the TALE protein including the N-terminal domain and C-terminal domain (including the half domain))
SEQ ID NO: 96. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPAQVVAIASNG
GGKQALETVQRLLPVLCQDHGLTPAQVVAIASNIGGKQALETVQRLLPVLCQA
HGLTPDQVVAIASNNGGKQALETVQRLLPVLCQAHGLTPAQVVAIASNNGGK
QALETVQRLLPVLCQAHGLTPDQVVAIASNNGGKQALETVQRLLPVLCQDHG
LTPEQVVAIASNNGGKQALETVQRLLPVLCQAHGLTPAQVVAIASHDGGKQAL
ETVQRLLPVLCQDHGLTPAQVVAIASNGGGKQALETVQRLLPVLCQDHGLTPA
QVVAIASHDGGKQALETVQRLLPVLCQAHGLTPDQVVAIASNGGGKQALETV
QRLLPVLCQAHGLTPAQVVAIASNGGGKQALETVQRLLPVLCQAHGLTPEQVV
AIASNGGGKQALETVQRLLPVLCQAHGLTPAQVVAIASNNGGKQALETVQRL
LPVLCQAHGLTPAQVVAIASNNGGKQALETVQRLLPVLCQAHGLTPEQVVAIA
SNGGGKQALETVQRLLPVLCQAHGLTPAQVVAIASNNGGKQALETVQRLLPV
LCQDHGLTPEQVVAIASNGGGKQALESIVAQLSRPDPALAALTNDHLVALACLG
GRPALDAVKKGLLVGSAIPVKRGATGETKVFTGNSNSPKSPTKGGCSGSETPGT
SESATPESSEVEFSHEYWMRHALTLAKRARDEREVPVGAVLVLNNRVIGEGWN
 
RAIGLHDPTAHAEIMALRQGGLVMQNYRLIDATLYVTFEPCVMCAGAMIHSRI
GRVVFGVRNSKRGAAGSLMNVLNYPGMNHRVEITEGILADECAALLCDFYR
MPRQVFNAQKKAQSSIN
 (The underlined portion corresponds to the TALE protein including the N-terminal domain and C-terminal domain (including the half domain))
SEQ ID NO: 79. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPAQVVAIASNI
GGKQALETVQRLLPVLCQAHGLTPAQVVAIASNNGGKQALETVQRLLPVLCQ
DHGLTPAQVVAIASNNGGKQALETVQRLLPVLCQAHGLTPEQVVAIASNNGGK
QALETVQRLLPVLCQAHGLTPAQVVAIASNNGGKQALETVQRLLPVLCQDHG
LTPDQVVAIASHDGGKQALETVQRLLPVLCQDHGLTPEQVVAIASNGGGKQAL
ETVQRLLPVLCQAHGLTPDQVVAIASHDGGKQALETVQRLLPVLCQDHGLTPD
QVVAIASNGGGKQALETVQRLLPVLCQAHGLTPEQVVAIASNGGGKQALETV
QRLLPVLCQAHGLTPAQVVAIASNGGGKQALETVQRLLPVLCQAHGLTPDQV
VAIASNNGGKQALETVQRLLPVLCQDHGLTPDQVVAIASNNGGKQALETVQR
LLPVLCQAHGLTPDQVVAIASNGGGKQALETVQRLLPVLCQAHGLTPAQVVAI
ASNNGGKQALETVQRLLPVLCQAHGLTPAQVVAIASNIGGKQALETVQRLLPV
LCQAHGLTPEQVVAIASNGGGKQALESIVAQLSRPDPALAALTNDHLVALACLG
GRPALDAVKKGLLVGSAIPVKRGATGETKVFTGNSNSPKSPTKGGCSGSETPGT
SESATPESSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGVRNSKRGAAGSLMNVLNYPGMNHRVEITEGILADECAALLCDFYR
MPRQVFNAQKKAQSSIN
 (The underlined portion corresponds to the TALE protein including the N-terminal domain and C-terminal domain (including the half domain))
SEQ ID NO: 98. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKYHGARALEALLTVAGELR
GPPLQLDTGQLLKIAKRGGVTAVEAVHAWRNALTGAPLNLTPEQVVAIASNGG
GKQALETVQRLLPVLCQAHGLTPDQVVAIASHDGGKQALETVQRLLPVLCQD
HGLTPDQVVAIASNGGGKQALETVQRLLPVLCQAHGLTPEQVVAIASNGGGK
QALETVQRLLPVLCQAHGLTPAQVVAIASNGGGKQALETVQRLLPVLCQAHG
LTPDQVVAIASNNGGKQALETVQRLLPVLCQDHGLTPEQVVAIASNNGGKQAL
ETVQRLLPVLCQAHGLTPDQVVAIASNGGGKQALETVQRLLPVLCQDHGLTPA
QVVAIASNNGGKQALETVQRLLPVLCQAHGLTPAQVVAIASNIGGKQALETVQ
RLLPVLCQDHGLTPDQVVAIASNIGGKQALETVQRLLPVLCQDHGLTPDQVVA
IASNNGGKQALETVQRLLPVLCQAHGLTPDQVVAIASNIGGKQALETVQRLLP
VLCQDHGLTPAQVVAIASNNGGKQALETVQRLLPVLCQDHGLTPEQVVAIASN
GGGKQALETVQRLLPVLCQAHGLTPDQVVAIASNGGGKQALETVQRLLPVLC
QAHGLTPEQVVAIASNGGGKQALESIVAQLSRPDPALAALTNDHLVALACLGG
RPALDAVKKGLLVGSAIPVKRGATGETKVFTGNSNSPKSPTKGGCSGSETPGTS
ESATPESSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGVRNSKRGAAGSLMNVLNYPGMNHRVEITEGILADECAALLCDFYR
MPRQVFNAQKKAQSSIN
 (The underlined portion corresponds to the TALE protein including the N-terminal domain and C-terminal domain (including the half domain))
SEQ ID NO: 99. MASVLTPLLLRGLTGSARRLPVPRAKIHSLDYKDHDGDYKDHDIDYK 
DDDDKGIRIQDLRTLGYSQQQQEKIKPKVRSTVAQHHEALVGHGFTHAHIVAL
SQHPAALGTVAVKYQDMIAALPEATHEAIVGVGKYHGARALEALLTVAGELR
GPPLQLDTGQLLKIAKRGGVTAVEAVHAWRNALTGAPLNLTPDQVVAIASNGG
GKQALETVQRLLPVLCQAHGLTPAQVVAIASNGGGKQALETVQRLLPVLCQA
HGLTPEQVVAIASNGGGKQALETVQRLLPVLCQAHGLTPAQVVAIASNNGGKQ
ALETVQRLLPVLCQDHGLTPAQVVAIASNNGGKQALETVQRLLPVLCQDHGLT
PDQVVAIASNGGGKQALETVQRLLPVLCQAHGLTPEQVVAIASNNGGKQALET
VQRLLPVLCQAHGLTPDQVVAIASNIGGKQALETVQRLLPVLCQDHGLTPDQV
VAIASNIGGKQALETVQRLLPVLCQAHGLTPDQVVAIASNNGGKQALETVQRL
LPVLCQDHGLTPAQVVAIASNIGGKQALETVQRLLPVLCQAHGLTPEQVVAIAS
NNGGKQALETVQRLLPVLCQAHGLTPEQVVAIASNGGGKQALETVQRLLPVL
CQAHGLTPDQVVAIASNGGGKQALETVQRLLPVLCQAHGLTPEQVVAIASNGG
GKQALETVQRLLPVLCQAHGLTPDQVVAIASNGGGKQALETVQRLLPVLCQA
HGLTPEQVVAIASNGGGKQALESIVAQLSRPDPALAALTNDHLVALACLGGRPA
LDAVKKGLLVGSAIPVKRGATGETKVFTGNSNSPKSPTKGGCSGSETPGTSESA
TPESSEVEFSHEYWMRHALTLAKRARDEREVPVGAVLVLNNRVIGEGWNRAI
GLHDPTAHAEIMALRQGGLVMQNYRLIDATLYVTFEPCVMCAGAMIHSRIGRV
VFGVRNSKRGAAGSLMNVLNYPGMNHRVEITEGILADECAALLCDFYRMPRQ
VFNAQKKAQSSIN
 (The underlined portion corresponds to the TALE protein including the N-terminal domain and C-terminal domain (including the half domain))

.The combinations of the first fusion protein and the second fusion protein used in the base editors shown in FIG. 2 are as follows.

| Base Editor ID | First Fusion Protein | Second Fusion Protein |
|---|---|---|
| 17N + 56C | SEQ ID NO: 78 | SEQ ID NO: 90 |
| 17N + 57C | SEQ ID NO: 78 | SEQ ID NO: 91 |
| 18N + 244Cv | SEQ ID NO: 79 | SEQ ID NO: 92 |
| 18N + 246Cv | SEQ ID NO: 79 | SEQ ID NO: 93 |
| 18N + 247Cv | SEQ ID NO: 79 | SEQ ID NO: 94 |
| 18N + 56C | SEQ ID NO: 79 | SEQ ID NO: 90 |
| 18N + 57C | SEQ ID NO: 79 | SEQ ID NO: 91 |
| 229Nv+43C | SEQ ID NO: 80 | SEQ ID NO: 95 |
| 229Nv + 56C | SEQ ID NO: 80 | SEQ ID NO: 90 |
| 229Nv + 57C | SEQ ID NO: 80 | SEQ ID NO: 91 |
| 231Nv + 48C | SEQ ID NO: 81 | SEQ ID NO: 96 |
| 231Nv + 57C | SEQ ID NO: 81 | SEQ ID NO: 91 |
| 232Nv + 53C | SEQ ID NO: 82 | SEQ ID NO: 97 |
| 232Nv + 56C | SEQ ID NO: 82 | SEQ ID NO: 90 |
| 17C + 248Nv | SEQ ID NO: 83 | SEQ ID NO: 88 |
| 17C + 57N | SEQ ID NO: 83 | SEQ ID NO: 87 |
| 17C + 249Nv | SEQ ID NO: 83 | SEQ ID NO: 89 |
| 18C + 248Nv | SEQ ID NO: 84 | SEQ ID NO: 88 |
| 18C + 56N | SEQ ID NO: 84 | SEQ ID NO: 86 |
| 18C + 57N | SEQ ID NO: 84 | SEQ ID NO: 87 |
| 229Cv + 248Nv | SEQ ID NO: 85 | SEQ ID NO: 88 |

### Example 7: Construction of TALED and ZFD for correction of m.G11778A mutation

TALED and ZFD (ZF deaminase) were constructed so that the editing window containing the G11778A point mutation in mitochondrial DNA ND4 to be corrected would span 1 to 20 bp. The method for constructing TALED is as described in Example 4. To construct ZFD, the sequence encoding the zinc finger proteins that bind to the target site was codon-optimized for expression in humans, and the double-stranded DNA sequence was synthesized as a gBlock DNA fragment from IDT (Integrated DNA Technologies). Using the synthesized gBlock DNA fragment and the expression vector backbone (containing MTS, HA tag, NES, DddAₜₒₓ1397N or 1397C, and TadA8e) as templates, the DNA fragments required for Gibson assembly were amplified using PrimeSTAR^{®} GXL DNA polymerase (TAKARA) and purified using a PCR SV mini kit (GeneAll). The purified DNA fragments were assembled using the HiFi DNA Assembly Kit (NEB), and the transformation into competent DH5α (enzynomics) E. coli cells and confirmation of the base sequence by Sanger sequencing (Macrogen) were performed as described in Example 4.

The first fusion protein and the second fusion protein were linked in the following order: [MTS]-[tag]-[TALE protein]-[linker]-[DddAₜₒₓ split], [MTS]-[tag]-[TALE protein]-[linker]-[DddAₜₒₓ split]-[linker]-[TadA8e], [MTS]-[tag]-[NES]-[linker]-[DddAₜₒₓ split]-[linker]-[ZF protein], or [MTS]-[tag]-[NES]-[linker]-[DddAₜₒₓ split]-[linker]-[TadA8e]-[linker]-[ZF protein]. As tags, 3X HA (SEQ ID NO: 136) or 3X FLAG (SEQ ID NO: 137) was used for fusion proteins containing TALE proteins, and 1X HA (SEQ ID NO: 138) was used for fusion proteins containing ZF proteins. The proteins used are located between the CMV promoter and the T7 promoter and terminator sequence.

### Example 8: Transfection of urine-derived cells from a patient carrying the G11778A mutation in the mitochondrial ND4 gene with a fusion protein

Cells were isolated from the urine of an LHON patient carrying the G11778A point mutation in the mitochondrial ND4 gene to obtain primary cells, which were aliquoted at passage 2 and cryopreserve.

The method for culturing urine-derived cells (UDC) from an LHON patient with the G11778A point mutation is as described in Example 5. Plasmids encoding the first fusion protein and the second fusion protein, 1 µg each for a total of 2 µg, were transfected into UDC cells (1.0 × 10⁴ cells) using the NEON 10 µL TRANSFECTION KIT (Invitrogen) and the NEON Transfection System (Invitrogen) by electroporation (1350V, 30ms, 1pulse). The transfected UDC cells were placed into 8-well Clear TC-Treated Multiple Well Plates (Corning) pre-coated with 0.1% gelatin and pre-filled with culture medium. Transfected cells were maintained at 37°C in 5% CO₂, with replacement of the culture medium. The cells were harvested after 6 days, the culture medium was removed, and the cells were lysed, and the process is the same as described in Example 5.

### Example 9: Sequencing And Base Editing Efficiency Measurement To Confirm Correction of m.g11778a Mutation

The reaction product obtained in Example 8 was used as a template without purification, and the sequence was analyzed using a targeted deep sequencing technique to analyze the base editing ratio of the target site. The method from library preparation to deep sequencing was as described in Example 6.

To screen the efficiency of the developed base editors in correcting m.A11778G, combinations of TALED and TALED, hybrid combinations of TALED and ZFD, and combinations of ZFD and ZFD were transfected. The DNA binding sites of 42 base editor combinations with high base editing efficiency among the above combinations and the efficiencies of m.A11778G correction were shown in FIG. 3.

The amino acid sequences of the first fusion protein and the second fusion protein used in the combinations of 42 base editors confirmed to have high base editing efficiency are as follows.

### First Fusion Protein

SEQ ID NO: 100. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPDQVV
AIASNIGGKQALETVQRLLPVLCQAHGLTPAQVVAIASNIGGKQALETVQRLLP
VLCQDHGLTPAQVVAIASNIGGKQALETVQRLLPVLCQAHGLTPAQVVAIASH
DGGKQALETVQRLLPVLCQDHGLTPAQVVAIASNGGGKQALETVQRLLPVLC
QDHGLTPAQVVAIASHDGGKQALETVQRLLPVLCQAHGLTPDQVVAIASNIGG
KQALETVQRLLPVLCQAHGLTPAQVVAIASNIGGKQALETVQRLLPVLCQDHG
LTPAQVVAIASNIGGKQALETVQRLLPVLCQAHGLTPDQVVAIASHDGGKQAL
ETVQRLLPVLCQAHGLTPDQVVAIASNGGGKQALETVQRLLPVLCQDHGLTPA
QVVAIASNIGGKQALETVQRLLPVLCQDHGLTPEQVVAIASHDGGKQALETVQ
RLLPVLCQAHGLTPAQVVAIASNNGGKQALETVQRLLPVLCQAHGLTPAQVVA
IASNIGGKQALETVQRLLPVLCQDHGLTPDQVVAIASNIGGKQALETVQRLLPV
LCQDHGLTPDQVVAIASHDGGKQALETVQRLLPVLCQAHGLTPAQVVAIASNN
GGKQALETVQRLLPVLCQDHGLTPEQVVAIASNGGGKQALESIVAQLSRPDPA
LAALTNDHLVALACLGGRPALDAVKKGLGGSGSGSYALGPYQISAPQLPAYNG
QTVGTFYYVNDAGGLESKVFSSGGPTPYPNYANAGHVEGQSALFMRDNGISE
GLVFHNNPEGTCGFCVNMTETLLPENAKMTVVPPEG
 (The underlined portion corresponds to the TALE protein including the N-terminal domain and C-terminal domain (including the half domain))
SEQ ID NO: 101. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
 
TVAGELRGPPLQLDTGQLLKIAKRGGVTAVEAVHAWRNALTGAPLNLTPDQVV
AIASNIGGKQALETVQRLLPVLCQAHGLTPDQVVAIASNIGGKQALETVQRLLP
VLCQAHGLTPAQVVAIASHDGGKQALETVQRLLPVLCQAHGLTPAQVVAIASN
GGGKQALETVQRLLPVLCQDHGLTPEQVVAIASHDGGKQALETVQRLLPVLC
QAHGLTPAQVVAIASNIGGKQALETVQRLLPVLCQDHGLTPDQVVAIASNIGGK
QALETVQRLLPVLCQAHGLTPDQVVAIASNIGGKQALETVQRLLPVLCQAHGL
TPAQVVAIASHDGGKQALETVQRLLPVLCQAHGLTPEQVVAIASNGGGKQALE
TVQRLLPVLCQAHGLTPDQVVAIASNIGGKQALETVQRLLPVLCQAHGLTPAQ
VVAIASHDGGKQALETVQRLLPVLCQDHGLTPEQVVAIASNNGGKQALETVQ
RLLPVLCQAHGLTPDQVVAIASNIGGKQALETVQRLLPVLCQDHGLTPDQVVA
IASNIGGKQALETVQRLLPVLCQAHGLTPAQVVAIASHDGGKQALETVQRLLP
VLCQAHGLTPDQVVAIASNNGGKQALETVQRLLPVLCQDHGLTPDQVVAIASH
DGGKQALETVQRLLPVLCQDHGLTPEQVVAIASNGGGKQALESIVAQLSRPDP
ALAALTNDHLVALACLGGRPALDAVKKGLGGSGSGSYALGPYQISAPQLPAYN
GQTVGTFYYVNDAGGLESKVFSSGGPTPYPNYANAGHVEGQSALFMRDNGIS
EGLVFHNNPEGTCGFCVNMTETLLPENAKMTVVPPEG
 (The underlined portion corresponds to the TALE protein including the N-terminal domain and C-terminal domain (including the half domain))
SEQ ID NO: 102. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPEQVV
AIASNIGGKQALETVQRLLPVLCQAHGLTPDQVVAIASHDGGKQALETVQRLL
PVLCQAHGLTPDQVVAIASNGGGKQALETVQRLLPVLCQDHGLTPAQVVAIAS
 
HDGGKQALETVQRLLPVLCQDHGLTPEQVVAIASNIGGKQALETVQRLLPVLC
QAHGLTPAQVVAIASNIGGKQALETVQRLLPVLCQDHGLTPEQVVAIASNIGGK
QALETVQRLLPVLCQAHGLTPDQVVAIASHDGGKQALETVQRLLPVLCQAHG
LTPDQVVAIASNGGGKQALETVQRLLPVLCQAHGLTPDQVVAIASNIGGKQAL
ETVQRLLPVLCQDHGLTPDQVVAIASHDGGKQALETVQRLLPVLCQAHGLTPA
QVVAIASNNGGKQALETVQRLLPVLCQDHGLTPDQVVAIASNIGGKQALETVQ
RLLPVLCQAHGLTPDQVVAIASNIGGKQALETVQRLLPVLCQAHGLTPAQVVAI
ASHDGGKQALETVQRLLPVLCQAHGLTPAQVVAIASNNGGKQALETVQRLLP
VLCQAHGLTPDQVVAIASHDGGKQALETVQRLLPVLCQDHGLTPAQVVAIASN
IGGKQALETVQRLLPVLCQAHGLTPEQVVAIASNGGGKQALESIVAQLSRPDPA
LAALTNDHLVALACLGGRPALDAVKKGLGGSGSGSYALGPYQISAPQLPAYNG
QTVGTFYYVNDAGGLESKVFSSGGPTPYPNYANAGHVEGQSALFMRDNGISE
GLVFHNNPEGTCGFCVNMTETLLPENAKMTVVPPEG
 (The underlined portion corresponds to the TALE protein including the N-terminal domain and C-terminal domain (including the half domain))
SEQ ID NO: 103. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPAQVV
AIASHDGGKQALETVQRLLPVLCQDHGLTPAQVVAIASNGGGKQALETVQRL
LPVLCQDHGLTPAQVVAIASHDGGKQALETVQRLLPVLCQAHGLTPDQVVAIA
SNIGGKQALETVQRLLPVLCQAHGLTPAQVVAIASNIGGKQALETVQRLLPVL
CQDHGLTPAQVVAIASNIGGKQALETVQRLLPVLCQAHGLTPDQVVAIASHDG
GKQALETVQRLLPVLCQAHGLTPDQVVAIASNGGGKQALETVQRLLPVLCQD
 
HGLTPAQVVAIASNIGGKQALETVQRLLPVLCQAHGLTPEQVVAIASHDGGKQ
ALETVQRLLPVLCQAHGLTPAQVVAIASNNGGKQALETVQRLLPVLCQAHGLT
PAQVVAIASNIGGKQALETVQRLLPVLCQDHGLTPDQVVAIASNIGGKQALETV
QRLLPVLCQDHGLTPDQVVAIASHDGGKQALETVQRLLPVLCQAHGLTPAQV
VAIASNNGGKQALETVQRLLPVLCQDHGLTPDQVVAIASHDGGKQALETVQR
LLPVLCQAHGLTPDQVVAIASNIGGKQALETVQRLLPVLCQAHGLTPAQVVAIA
SHDGGKQALETVQRLLPVLCQAHGLTPEQVVAIASNGGGKQALESIVAQLSRP
DPALAALTNDHLVALACLGGRPALDAVKKGLGGSGSGSYALGPYQISAPQLPA
YNGQTVGTFYYVNDAGGLESKVFSSGGPTPYPNYANAGHVEGQSALFMRDN
GISEGLVFHNNPEGTCGFCVNMTETLLPENAKMTVVPPEG
 (The underlined portion corresponds to the TALE protein including the N-terminal domain and C-terminal domain (including the half domain))
SEQ ID NO: 104. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPAQVV
AIASNGGGKQALETVQRLLPVLCQDHGLTPEQVVAIASHDGGKQALETVQRLL
PVLCQAHGLTPAQVVAIASNIGGKQALETVQRLLPVLCQDHGLTPDQVVAIAS
NIGGKQALETVQRLLPVLCQAHGLTPDQVVAIASNIGGKQALETVQRLLPVLC
QAHGLTPAQVVAIASHDGGKQALETVQRLLPVLCQAHGLTPEQVVAIASNGGG
KQALETVQRLLPVLCQAHGLTPDQVVAIASNIGGKQALETVQRLLPVLCQAHG
LTPAQVVAIASHDGGKQALETVQRLLPVLCQAHGLTPEQVVAIASNNGGKQAL
ETVQRLLPVLCQAHGLTPDQVVAIASNIGGKQALETVQRLLPVLCQDHGLTPD
QVVAIASNIGGKQALETVQRLLPVLCQAHGLTPAQVVAIASHDGGKQALETVQ
 
RLLPVLCQAHGLTPDQVVAIASNNGGKQALETVQRLLPVLCQDHGLTPDQVV
AIASHDGGKQALETVQRLLPVLCQDHGLTPEQVVAIASNIGGKQALETVQRLL
PVLCQAHGLTPDQVVAIASHDGGKQALETVQRLLPVLCQAHGLTPDQVVAIAS
NGGGKQALETVQRLLPVLCQDHGLTPEQVVAIASNGGGKQALESIVAQLSRPD
PALAALTNDHLVALACLGGRPALDAVKKGLGGSGSGSYALGPYQISAPQLPAY
NGQTVGTFYYVNDAGGLESKVFSSGGPTPYPNYANAGHVEGQSALFMRDNGI
SEGLVFHNNPEGTCGFCVNMTETLLPENAKMTVVPPEG
 (The underlined portion corresponds to the TALE protein including the N-terminal domain and C-terminal domain (including the half domain))
SEQ ID NO: 105. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKQWSGARALEA
LLTVAGELRGPPLQLDTGQLLKIAKRGGVTAVEAVHAWRNALTGAPLNLTPAQ
VVAIASHDGGKQALETVQRLLPVLCQDHGLTPEQVVAIASNIGGKQALETVQR
LLPVLCQAHGLTPAQVVAIASNIGGKQALETVQRLLPVLCQDHGLTPEQVVAIA
SNIGGKQALETVQRLLPVLCQAHGLTPDQVVAIASHDGGKQALETVQRLLPVL
CQAHGLTPDQVVAIASNGGGKQALETVQRLLPVLCQDHGLTPDQVVAIASNIG
GKQALETVQRLLPVLCQDHGLTPDQVVAIASHDGGKQALETVQRLLPVLCQA
HGLTPAQVVAIASNNGGKQALETVQRLLPVLCQAHGLTPDQVVAIASNIGGKQ
ALETVQRLLPVLCQAHGLTPDQVVAIASNIGGKQALETVQRLLPVLCQAHGLT
PAQVVAIASHDGGKQALETVQRLLPVLCQAHGLTPAQVVAIASNNGGKQALET
VQRLLPVLCQAHGLTPDQVVAIASHDGGKQALETVQRLLPVLCQDHGLTPAQ
VVAIASNIGGKQALETVQRLLPVLCQAHGLTPAQVVAIASHDGGKQALETVQR
LLPVLCQDHGLTPAQVVAIASNGGGKQALETVQRLLPVLCQDHGLTPAQVVAI
 
ASHDGGKQALETVQRLLPVLCQAHGLTPEQVVAIASNGGGKQALESIVAQLSR
PDPALAALTNDHLVALACLGGRPALDAVKKGLGGSGSGSYALGPYQISAPQLP
AYNGQTVGTFYYVNDAGGLESKVFSSGGPTPYPNYANAGHVEGQSALFMRD
NGISEGLVFHNNPEGTCGFCVNMTETLLPENAKMTVVPPEG
 (The underlined portion corresponds to the TALE protein including the N-terminal domain and C-terminal domain (including the half domain))
SEQ ID NO: 106. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPDQVV
AIASNIGGKQALETVQRLLPVLCQAHGLTPAQVVAIASNIGGKQALETVQRLLP
VLCQDHGLTPAQVVAIASNIGGKQALETVQRLLPVLCQAHGLTPDQVVAIASH
DGGKQALETVQRLLPVLCQAHGLTPDQVVAIASNGGGKQALETVQRLLPVLC
QDHGLTPAQVVAIASNIGGKQALETVQRLLPVLCQDHGLTPEQVVAIASHDGG
KQALETVQRLLPVLCQAHGLTPAQVVAIASNNGGKQALETVQRLLPVLCQAH
GLTPAQVVAIASNIGGKQALETVQRLLPVLCQAHGLTPDQVVAIASNIGGKQAL
ETVQRLLPVLCQDHGLTPDQVVAIASHDGGKQALETVQRLLPVLCQAHGLTPA
QVVAIASNNGGKQALETVQRLLPVLCQDHGLTPDQVVAIASHDGGKQALETV
QRLLPVLCQAHGLTPDQVVAIASNIGGKQALETVQRLLPVLCQAHGLTPAQVV
AIASHDGGKQALETVQRLLPVLCQAHGLTPAQVVAIASNGGGKQALETVQRL
LPVLCQDHGLTPEQVVAIASHDGGKQALETVQRLLPVLCQAHGLTPAQVVAIA
SNIGGKQALETVQRLLPVLCQDHGLTPEQVVAIASNGGGKQALESIVAQLSRPD
PALAALTNDHLVALACLGGRPALDAVKKGLGGSGSGSYALGPYQISAPQLPAY
NGQTVGTFYYVNDAGGLESKVFSSGGPTPYPNYANAGHVEGQSALFMRDNGI
 SEGLVFHNNPEGTCGFCVNMTETLLPENAKMTVVPPEG (The underlined portion corresponds to the TALE protein including the N-terminal domain and C-terminal domain (including the half domain))
SEQ ID NO: 107. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPDQVV
AIASNIGGKQALETVQRLLPVLCQAHGLTPDQVVAIASNIGGKQALETVQRLLP
VLCQAHGLTPAQVVAIASHDGGKQALETVQRLLPVLCQAHGLTPEQVVAIASN
GGGKQALETVQRLLPVLCQAHGLTPDQVVAIASNIGGKQALETVQRLLPVLCQ
AHGLTPAQVVAIASHDGGKQALETVQRLLPVLCQDHGLTPEQVVAIASNNGGK
QALETVQRLLPVLCQAHGLTPDQVVAIASNIGGKQALETVQRLLPVLCQDHGL
TPDQVVAIASNIGGKQALETVQRLLPVLCQAHGLTPAQVVAIASHDGGKQALE
TVQRLLPVLCQAHGLTPDQVVAIASNNGGKQALETVQRLLPVLCQDHGLTPD
QVVAIASHDGGKQALETVQRLLPVLCQDHGLTPEQVVAIASNIGGKQALETVQ
RLLPVLCQAHGLTPDQVVAIASHDGGKQALETVQRLLPVLCQAHGLTPDQVV
AIASNGGGKQALETVQRLLPVLCQDHGLTPDQVVAIASHDGGKQALETVQRL
LPVLCQAHGLTPDQVVAIASNIGGKQALETVQRLLPVLCQAHGLTPAQVVAIAS
HDGGKQALETVQRLLPVLCQAHGLTPEQVVAIASNGGGKQALESIVAQLSRPD
PALAALTNDHLVALACLGGRPALDAVKKGLGGSGSGSYALGPYQISAPQLPAY
NGQTVGTFYYVNDAGGLESKVFSSGGPTPYPNYANAGHVEGQSALFMRDNGI
SEGLVFHNNPEGTCGFCVNMTETLLPENAKMTVVPPEG
 (The underlined portion corresponds to the TALE protein including the N-terminal domain and C-terminal domain (including the half domain))
SEQ ID NO: 108. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPEQVV
AIASNIGGKQALETVQRLLPVLCQAHGLTPDQVVAIASHDGGKQALETVQRLL
PVLCQAHGLTPDQVVAIASNGGGKQALETVQRLLPVLCQDHGLTPDQVVAIAS
NIGGKQALETVQRLLPVLCQDHGLTPDQVVAIASHDGGKQALETVQRLLPVLC
QAHGLTPAQVVAIASNNGGKQALETVQRLLPVLCQDHGLTPDQVVAIASNIGG
KQALETVQRLLPVLCQAHGLTPDQVVAIASNIGGKQALETVQRLLPVLCQAHG
LTPAQVVAIASHDGGKQALETVQRLLPVLCQAHGLTPAQVVAIASNNGGKQAL
ETVQRLLPVLCQAHGLTPDQVVAIASHDGGKQALETVQRLLPVLCQDHGLTPA
QVVAIASNIGGKQALETVQRLLPVLCQAHGLTPAQVVAIASHDGGKQALETVQ
RLLPVLCQDHGLTPAQVVAIASNGGGKQALETVQRLLPVLCQDHGLTPAQVVA
IASHDGGKQALETVQRLLPVLCQAHGLTPDQVVAIASNIGGKQALETVQRLLP
VLCQDHGLTPDQVVAIASHDGGKQALETVQRLLPVLCQDHGLTPAQVVAIASN
IGGKQALETVQRLLPVLCQAHGLTPEQVVAIASNGGGKQALESIVAQLSRPDPA
LAALTNDHLVALACLGGRPALDAVKKGLGGSGSGSYALGPYQISAPQLPAYNG
QTVGTFYYVNDAGGLESKVFSSGGPTPYPNYANAGHVEGQSALFMRDNGISE
GLVFHNNPEGTCGFCVNMTETLLPENAKMTVVPPEG
 (The underlined portion corresponds to the TALE protein including the N-terminal domain and C-terminal domain (including the half domain))
SEQ ID NO: 109. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
 
SQHPAALGTVAVKYQDMIAALPEATHEAIVGVGKYHGARALEALLTVAGELR
GPPLQLDTGQLLKIAKRGGVTAVEAVHAWRNALTGAPLNLTPAQVVAIASNGG
GKQALETVQRLLPVLCQDHGLTPEQVVAIASHDGGKQALETVQRLLPVLCQA
HGLTPAQVVAIASNIGGKQALETVQRLLPVLCQDHGLTPDQVVAIASNIGGKQA
LETVQRLLPVLCQAHGLTPDQVVAIASNIGGKQALETVQRLLPVLCQAHGLTP
AQVVAIASHDGGKQALETVQRLLPVLCQAHGLTPEQVVAIASNGGGKQALET
VQRLLPVLCQAHGLTPDQVVAIASNIGGKQALETVQRLLPVLCQAHGLTPAQV
VAIASHDGGKQALETVQRLLPVLCQAHGLTPEQVVAIASNNGGKQALETVQRL
LPVLCQAHGLTPDQVVAIASNIGGKQALETVQRLLPVLCQDHGLTPDQVVAIA
SNIGGKQALETVQRLLPVLCQAHGLTPAQVVAIASHDGGKQALETVQRLLPVL
CQAHGLTPDQVVAIASNNGGKQALETVQRLLPVLCQDHGLTPDQVVAIASHD
GGKQALETVQRLLPVLCQDHGLTPEQVVAIASNIGGKQALETVQRLLPVLCQA
HGLTPDQVVAIASHDGGKQALETVQRLLPVLCQAHGLTPDQVVAIASNGGGK
QALETVQRLLPVLCQDHGLTPEQVVAIASNGGGKQALESIVAQLSRPDPALAA
LTNDHLVALACLGGRPALDAVKKGLLVGSAIPVKRGATGETKVFTGNSNSPKSP
TKGGCSGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEP
CVMCAGAMIHSRIGRVVFGVRNSKRGAAGSLMNVLNYPGMNHRVEITEGILA
DECAALLCDFYRMPRQVFNAQKKAQSSIN
 (The underlined portion corresponds to the TALE protein including the N-terminal domain and C-terminal domain (including the half domain))
SEQ ID NO: 110. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKQWSGARALEALLTVAGEL
 
RGPPLQLDTGQLLKIAKRGGVTAVEAVHAWRNALTGAPLNLTPAQVVAIASHD
GGKQALETVQRLLPVLCQDHGLTPEQVVAIASNIGGKQALETVQRLLPVLCQA
HGLTPAQVVAIASNIGGKQALETVQRLLPVLCQDHGLTPEQVVAIASNIGGKQA
LETVQRLLPVLCQAHGLTPDQVVAIASHDGGKQALETVQRLLPVLCQAHGLTP
DQVVAIASNGGGKQALETVQRLLPVLCQDHGLTPDQVVAIASNIGGKQALETV
QRLLPVLCQDHGLTPDQVVAIASHDGGKQALETVQRLLPVLCQAHGLTPAQV
VAIASNNGGKQALETVQRLLPVLCQAHGLTPDQVVAIASNIGGKQALETVQRL
LPVLCQAHGLTPDQVVAIASNIGGKQALETVQRLLPVLCQAHGLTPAQVVAIAS
HDGGKQALETVQRLLPVLCQAHGLTPAQVVAIASNNGGKQALETVQRLLPVL
CQAHGLTPDQVVAIASHDGGKQALETVQRLLPVLCQDHGLTPAQVVAIASNIG
GKQALETVQRLLPVLCQAHGLTPAQVVAIASHDGGKQALETVQRLLPVLCQD
HGLTPAQVVAIASNGGGKQALETVQRLLPVLCQDHGLTPAQVVAIASHDGGKQ
ALETVQRLLPVLCQAHGLTPEQVVAIASNGGGKQALESIVAQLSRPDPALAALT
NDHLVALACLGGRPALDAVKKGLLVGSAIPVKRGATGETKVFTGNSNSPKSPT
KGGCSGSETPGTSESATPESSEVEFSHEYWMRHALTLAKRARDEREVPVGAVL
VLNNRVIGEGWNRAIGLHDPTAHAEIMALRQGGLVMQNYRLIDATLYVTFEPC
VMCAGAMIHSRIGRVVFGVRNSKRGAAGSLMNVLNYPGMNHRVEITEGILAD
ECAALLCDFYRMPRQVFNAQKKAQSSIN
 (The underlined portion corresponds to the TALE protein including the N-terminal domain and C-terminal domain (including the half domain))
SEQ ID NO: 111. 
MLGFVGRVAAAPASGALRRLTPSASLPPAQLLLRAAPTAVHPVRDYAA
QYPYDVPDYAVDEMTKKFGTLTIHDTEKAAEFGIHGVPAAMGGSYALGPYQIS
APQLPAYNGQTVGTFYYVNDAGGLESKVFSSGGPTPYPNYANAGHVEGQSAL
FMRDNGISEGLVFHNNPEGTCGFCVNMTETLLPENAKMTVVPPEGSGTPHEV
 
GVYTLSGTPHEVGVYTLYKCPECGKSFSSKKALTEHQRTHTGEKPYKCPECGK
SFSTHLDLIRHQRTHTGEKPYKCPECGKSFSHTGHLLEHQRTHTGEKPFECKDC
GKAFIQKSNLIRHQRTH
 (The underlined portion corresponds to the zinc finger protein)
SEQ ID NO: 112. 
MLGFVGRVAAAPASGALRRLTPSASLPPAQLLLRAAPTAVHPVRDYAA
QYPYDVPDYAVDEMTKKFGTLTIHDTEKAAEFGIHGVPAAMGGSYALGPYQIS
APQLPAYNGQTVGTFYYVNDAGGLESKVFSSGGPTPYPNYANAGHVEGQSAL
FMRDNGISEGLVFHNNPEGTCGFCVNMTETLLPENAKMTVVPPEGSGTPHEV
GVYTLSGTPHEVGVYTLYSCGICGKSFSDSSAKRRHCILHTGEKPYKCPECGKS
FSSPADLTRHQRTHLRQKDGERPYKCPECGKSFSTHLDLIRHQRTHTGEKPYKC
PECGKSFSHTGHLLEHQRTHTGEKPFECKDCGKAFIQKSNLIRHQRTH
 (The underlined portion corresponds to the zinc finger protein)
SEQ ID NO: 113. 
MLGFVGRVAAAPASGALRRLTPSASLPPAQLLLRAAPTAVHPVRDYAA
QYPYDVPDYAVDEMTKKFGTLTIHDTEKAAEFGIHGVPAAMGGSYALGPYQIS
APQLPAYNGQTVGTFYYVNDAGGLESKVFSSGGPTPYPNYANAGHVEGQSAL
FMRDNGISEGLVFHNNPEGTCGFCVNMTETLLPENAKMTVVPPEGSGTPHEV
GVYTLSGTPHEVGVYTLYKCPECGKSFSTHLDLIRHQRTHTGEKPYKCPECGK
SFSHTGHLLEHQRTHTGEKPFECKDCGKAFIQKSNLIRHQRTHLRQKDGGGSE
RPYKCPECGKSFSTHLDLIRHQRTHTGEKPYKCDECGKNFTQSSNLIVHKRIHT
GEKPYKCPECGKSFSTHLDLIRHQRTH
 (The underlined portion corresponds to the zinc finger protein)
SEQ ID NO: 114. 
MLGFVGRVAAAPASGALRRLTPSASLPPAQLLLRAAPTAVHPVRDYAA
QYPYDVPDYAVDEMTKKFGTLTIHDTEKAAEFGIHGVPAAMGGSAIPVKRGAT
 
GETKVFTGNSNSPKSPTKGGCSGSETPGTSESATPESSEVEFSHEYWMRHALTL
AKRARDEREVPVGAVLVLNNRVIGEGWNRAIGLHDPTAHAEIMALRQGGLVM
QNYRLIDATLYVTFEPCVMCAGAMIHSRIGRVVFGVRNSKRGAAGSLMNVLN
YPGMNHRVEITEGILADECAALLCDFYRMPRQVFNAQKKAQSSINSGTPHEVG
VYTLSGTPHEVGVYTLYKCPECGKSFSTHLDLIRHQRTHTGEKPYKCPECGKS
FSHTGHLLEHQRTHTGEKPFECKDCGKAFIQKSNLIRHQRTHLRQKDGGGSER
PYKCPECGKSFSTHLDLIRHQRTHTGEKPYKCDECGKNFTQSSNLIVHKRIHTG
EKPYKCPECGKSFSTHLDLIRHQRTH
 (The underlined portion corresponds to the zinc finger protein)

### Second Fusion Protein

SEQ ID NO: 115. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPAQVV
AIASNIGGKQALETVQRLLPVLCQAHGLTPEQVVAIASNNGGKQALETVQRLL
PVLCQAHGLTPDQVVAIASNGGGKQALETVQRLLPVLCQDHGLTPDQVVAIAS
HDGGKQALETVQRLLPVLCQAHGLTPAQVVAIASHDGGKQALETVQRLLPVL
CQDHGLTPAQVVAIASNGGGKQALETVQRLLPVLCQDHGLTPAQVVAIASNGG
GKQALETVQRLLPVLCQAHGLTPAQVVAIASNNGGKQALETVQRLLPVLCQD
HGLTPAQVVAIASNIGGKQALETVQRLLPVLCQAHGLTPDQVVAIASNNGGKQ
ALETVQRLLPVLCQDHGLTPAQVVAIASNIGGKQALETVQRLLPVLCQAHGLT
PEQVVAIASNNGGKQALETVQRLLPVLCQAHGLTPAQVVAIASNIGGKQALET
VQRLLPVLCQAHGLTPAQVVAIASNNGGKQALETVQRLLPVLCQDHGLTPAQ
VVAIASNNGGKQALETVQRLLPVLCQAHGLTPAQVVAIASNIGGKQALETVQR
 
LLPVLCQAHGLTPDQVVAIASNGGGKQALETVQRLLPVLCQAHGLTPDQVVAI
ASNGGGKQALETVQRLLPVLCQAHGLTPEQVVAIASNGGGKQALESIVAQLSR
PDPALAALTNDHLVALACLGGRPALDAVKKGLGGSGSGSYALGPYQISAPQLP
AYNGQTVGTFYYVNDAGGLESKVFSSGGPTPYPNYANAGHVEGQSALFMRD
NGISEGLVFHNNPEGTCGFCVNMTETLLPENAKMTVVPPEG
 (The underlined portion corresponds to the TALE protein including the N-terminal domain and C-terminal domain (including the half domain))
SEQ ID NO: 116. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPAQVV
AIASNIGGKQALETVQRLLPVLCQDHGLTPDQVVAIASNIGGKQALETVQRLLP
VLCQDHGLTPDQVVAIASNNGGKQALETVQRLLPVLCQAHGLTPAQVVAIASN
GGGKQALETVQRLLPVLCQDHGLTPAQVVAIASHDGGKQALETVQRLLPVLC
QAHGLTPEQVVAIASHDGGKQALETVQRLLPVLCQAHGLTPEQVVAIASNGGG
KQALETVQRLLPVLCQAHGLTPAQVVAIASNGGGKQALETVQRLLPVLCQAH
GLTPDQVVAIASNNGGKQALETVQRLLPVLCQAHGLTPDQVVAIASNIGGKQA
LETVQRLLPVLCQDHGLTPAQVVAIASNNGGKQALETVQRLLPVLCQDHGLTP
AQVVAIASNIGGKQALETVQRLLPVLCQAHGLTPDQVVAIASNNGGKQALETV
QRLLPVLCQDHGLTPAQVVAIASNIGGKQALETVQRLLPVLCQAHGLTPEQVV
AIASNNGGKQALETVQRLLPVLCQAHGLTPAQVVAIASNNGGKQALETVQRL
LPVLCQDHGLTPAQVVAIASNIGGKQALETVQRLLPVLCQAHGLTPEQVVAIAS
NGGGKQALETVQRLLPVLCQAHGLTPEQVVAIASNGGGKQALESIVAQLSRPD
PALAALTNDHLVALACLGGRPALDAVKKGLGGSGSGSYALGPYQISAPQLPAY
 
NGQTVGTFYYVNDAGGLESKVFSSGGPTPYPNYANAGHVEGQSALFMRDNGI
SEGLVFHNNPEGTCGFCVNMTETLLPENAKMTVVPPEG
 (The underlined portion corresponds to the TALE protein including the N-terminal domain and C-terminal domain (including the half domain))
SEQ ID NO: 117. 
MALSRAVCGTSRQLAPVLGYLGSRQKHSLPDYPYDVPDYAGYPYDVP
DYAGYPYDVPDYAGIRIQDLRTLGYSQQQQEKIKPKVRSTVAQHHEALVGHGF
THAHIVALSQHPAALGTVAVKYQDMIAALPEATHEAIVGVGKYHGARALEALL
TVAGELRGPPLQLDTGQLLKIAKRGGVTAVEAVHAWRNALTGAPLNLTPAQVV
AIASNNGGKQALETVQRLLPVLCQDHGLTPAQVVAIASNGGGKQALETVQRL
LPVLCQAHGLTPDQVVAIASHDGGKQALETVQRLLPVLCQDHGLTPAQVVAIA
SHDGGKQALETVQRLLPVLCQAHGLTPDQVVAIASNGGGKQALETVQRLLPV
LCQAHGLTPAQVVAIASNGGGKQALETVQRLLPVLCQDHGLTPDQVVAIASNN
GGKQALETVQRLLPVLCQDHGLTPAQVVAIASNIGGKQALETVQRLLPVLCQA
HGLTPEQVVAIASNNGGKQALETVQRLLPVLCQAHGLTPDQVVAIASNIGGKQ
ALETVQRLLPVLCQDHGLTPAQVVAIASNNGGKQALETVQRLLPVLCQDHGLT
PAQVVAIASNIGGKQALETVQRLLPVLCQAHGLTPAQVVAIASNNGGKQALET
VQRLLPVLCQAHGLTPDQVVAIASNNGGKQALETVQRLLPVLCQDHGLTPAQ
VVAIASNIGGKQALETVQRLLPVLCQAHGLTPEQVVAIASNGGGKQALETVQR
LLPVLCQAHGLTPDQVVAIASNGGGKQALETVQRLLPVLCQDHGLTPDQVVAI
ASNIGGKQALETVQRLLPVLCQDHGLTPEQVVAIASNGGGKQALESIVAQLSRP
DPALAALTNDHLVALACLGGRPALDAVKKGLGGSGSGSYALGPYQISAPQLPA
YNGQTVGTFYYVNDAGGLESKVFSSGGPTPYPNYANAGHVEGQSALFMRDN
GISEGLVFHNNPEGTCGFCVNMTETLLPENAKMTVVPPEG
 (The underlined portion corresponds to the TALE protein including the N-terminal domain and C-terminal domain (including the half domain))
SEQ ID NO: 118. 
MASVLTPLLLRGLTGSARRLPVPRAKIHSLDYKDHDGDYKDHDIDYK
DDDDKGIRIQDLRTLGYSQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPEQVVAIASNN
GGKQALETVQRLLPVLCQAHGLTPDQVVAIASNIGGKQALETVQRLLPVLCQD
HGLTPDQVVAIASNIGGKQALETVQRLLPVLCQAHGLTPAQVVAIASNNGGKQ
ALETVQRLLPVLCQDHGLTPAQVVAIASNGGGKQALETVQRLLPVLCQAHGLT
PDQVVAIASHDGGKQALETVQRLLPVLCQDHGLTPAQVVAIASHDGGKQALE
TVQRLLPVLCQAHGLTPDQVVAIASNGGGKQALETVQRLLPVLCQAHGLTPAQ
VVAIASNGGGKQALETVQRLLPVLCQAHGLTPDQVVAIASNNGGKQALETVQ
RLLPVLCQDHGLTPAQVVAIASNIGGKQALETVQRLLPVLCQAHGLTPEQVVAI
ASNNGGKQALETVQRLLPVLCQAHGLTPDQVVAIASNIGGKQALETVQRLLPV
LCQDHGLTPAQVVAIASNNGGKQALETVQRLLPVLCQDHGLTPAQVVAIASNI
GGKQALETVQRLLPVLCQAHGLTPAQVVAIASNNGGKQALETVQRLLPVLCQ
AHGLTPDQVVAIASNNGGKQALETVQRLLPVLCQDHGLTPAQVVAIASNIGGK
QALETVQRLLPVLCQAHGLTPEQVVAIASNGGGKQALESIVAQLSRPDPALAA
LTNDHLVALACLGGRPALDAVKKGLLVGSAIPVKRGATGETKVFTGNSNSPKSP
TKGGCSGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEP
CVMCAGAMIHSRIGRVVFGVRNSKRGAAGSLMNVLNYPGMNHRVEITEGILA
DECAALLCDFYRMPRQVFNAQKKAQSSIN
 (The underlined portion corresponds to the TALE protein including the N-terminal domain and C-terminal domain (including the half domain))
SEQ ID NO: 119. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKYHGARALEALLTVAGELR
GPPLQLDTGQLLKIAKRGGVTAVEAVHAWRNALTGAPLNLTPAQVVAIASNIG
GKQALETVQRLLPVLCQDHGLTPDQVVAIASNIGGKQALETVQRLLPVLCQDH
GLTPDQVVAIASNNGGKQALETVQRLLPVLCQAHGLTPAQVVAIASNGGGKQ
ALETVQRLLPVLCQDHGLTPAQVVAIASHDGGKQALETVQRLLPVLCQAHGLT
PEQVVAIASHDGGKQALETVQRLLPVLCQAHGLTPEQVVAIASNGGGKQALET
VQRLLPVLCQAHGLTPAQVVAIASNGGGKQALETVQRLLPVLCQAHGLTPDQ
VVAIASNNGGKQALETVQRLLPVLCQAHGLTPDQVVAIASNIGGKQALETVQR
LLPVLCQDHGLTPAQVVAIASNNGGKQALETVQRLLPVLCQDHGLTPAQVVAI
ASNIGGKQALETVQRLLPVLCQAHGLTPDQVVAIASNNGGKQALETVQRLLPV
LCQDHGLTPAQVVAIASNIGGKQALETVQRLLPVLCQAHGLTPEQVVAIASNN
GGKQALETVQRLLPVLCQAHGLTPAQVVAIASNNGGKQALETVQRLLPVLCQ
DHGLTPAQVVAIASNIGGKQALETVQRLLPVLCQAHGLTPEQVVAIASNGGGK
QALETVQRLLPVLCQAHGLTPEQVVAIASNGGGKQALESIVAQLSRPDPALAA
LTNDHLVALACLGGRPALDAVKKGLLVGSAIPVKRGATGETKVFTGNSNSPKSP
TKGGCSGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEP
CVMCAGAMIHSRIGRVVFGVRNSKRGAAGSLMNVLNYPGMNHRVEITEGILA
DECAALLCDFYRMPRQVFNAQKKAQSSIN
 (The underlined portion corresponds to the TALE protein including the N-terminal domain and C-terminal domain (including the half domain))
SEQ ID NO: 120. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKYHGARALEALLTVAGELR
GPPLQLDTGQLLKIAKRGGVTAVEAVHAWRNALTGAPLNLTPAQVVAIASNGG
GKQALETVQRLLPVLCQDHGLTPAQVVAIASHDGGKQALETVQRLLPVLCQA
HGLTPEQVVAIASHDGGKQALETVQRLLPVLCQAHGLTPEQVVAIASNGGGKQ
ALETVQRLLPVLCQAHGLTPAQVVAIASNGGGKQALETVQRLLPVLCQAHGLT
PDQVVAIASNNGGKQALETVQRLLPVLCQDHGLTPDQVVAIASNIGGKQALET
VQRLLPVLCQDHGLTPAQVVAIASNNGGKQALETVQRLLPVLCQDHGLTPAQ
VVAIASNIGGKQALETVQRLLPVLCQAHGLTPDQVVAIASNNGGKQALETVQR
LLPVLCQDHGLTPAQVVAIASNIGGKQALETVQRLLPVLCQAHGLTPEQVVAIA
SNNGGKQALETVQRLLPVLCQAHGLTPAQVVAIASNNGGKQALETVQRLLPV
LCQDHGLTPAQVVAIASNIGGKQALETVQRLLPVLCQAHGLTPEQVVAIASNG
GGKQALETVQRLLPVLCQAHGLTPDQVVAIASNGGGKQALETVQRLLPVLCQ
AHGLTPDQVVAIASNIGGKQALETVQRLLPVLCQDHGLTPDQVVAIASNGGGK
QALETVQRLLPVLCQAHGLTPEQVVAIASNGGGKQALESIVAQLSRPDPALAA
LTNDHLVALACLGGRPALDAVKKGLLVGSAIPVKRGATGETKVFTGNSNSPKSP
TKGGCSGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEP
CVMCAGAMIHSRIGRVVFGVRNSKRGAAGSLMNVLNYPGMNHRVEITEGILA
DECAALLCDFYRMPRQVFNAQKKAQSSIN
 (The underlined portion corresponds to the TALE protein including the N-terminal domain and C-terminal domain (including the half domain))
SEQ ID NO: 121. MASVLTPLLLRGLTGSARRLPVPRAKIHSLDYKDHDGDYKDHDIDYK 
DDDDKGIRIQDLRTLGYSQQQQEKIKPKVRSTVAQHHEALVGHGFTHAHIVAL
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPAQVVAIASNG
GGKQALETVQRLLPVLCQAHGLTPAQVVAIASNNGGKQALETVQRLLPVLCQ
DHGLTPAQVVAIASNIGGKQALETVQRLLPVLCQDHGLTPAQVVAIASNIGGKQ
ALETVQRLLPVLCQAHGLTPEQVVAIASNNGGKQALETVQRLLPVLCQAHGLT
PDQVVAIASNGGGKQALETVQRLLPVLCQDHGLTPDQVVAIASHDGGKQALE
TVQRLLPVLCQAHGLTPAQVVAIASHDGGKQALETVQRLLPVLCQDHGLTPAQ
VVAIASNGGGKQALETVQRLLPVLCQAHGLTPAQVVAIASNGGGKQALETVQ
RLLPVLCQAHGLTPAQVVAIASNNGGKQALETVQRLLPVLCQDHGLTPAQVVA
IASNIGGKQALETVQRLLPVLCQDHGLTPDQVVAIASNNGGKQALETVQRLLP
VLCQDHGLTPAQVVAIASNIGGKQALETVQRLLPVLCQAHGLTPEQVVAIASN
NGGKQALETVQRLLPVLCQAHGLTPAQVVAIASNIGGKQALETVQRLLPVLCQ
AHGLTPAQVVAIASNNGGKQALETVQRLLPVLCQDHGLTPAQVVAIASNNGGK
QALETVQRLLPVLCQAHGLTPEQVVAIASNGGGKQALESIVAQLSRPDPALAA
LTNDHLVALACLGGRPALDAVKKGLLVGSAIPVKRGATGETKVFTGNSNSPKSP
TKGGCSGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEP
CVMCAGAMIHSRIGRVVFGVRNSKRGAAGSLMNVLNYPGMNHRVEITEGILA
DECAALLCDFYRMPRQVFNAQKKAQSSIN
 (The underlined portion corresponds to the TALE protein including the N-terminal domain and C-terminal domain (including the half domain))
SEQ ID NO: 122. 
MASVLTPLLLRGLTGSARRLPVPRAKIHSLDYKDHDGDYKDHDIDYK
DDDDKGIRIQDLRTLGYSQQQEKIKPKVRSTVAQHHEALVGHGFTHAHIVAL
 
SQHPAALGTVAVKYQDMIAALPEATHEAIVGVGKQWSGARALEALLTVAGEL
RGPPLQLDTGQLLKIAKRGGVTAVEAVHAWRNALTGAPLNLTPEQVVAIASNG
GGKQALETVQRLLPVLCQAHGLTPAQVVAIASNGGGKQALETVQRLLPVLCQ
AHGLTPDQVVAIASNNGGKQALETVQRLLPVLCQDHGLTPAQVVAIASNIGGK
QALETVQRLLPVLCQDHGLTPDQVVAIASNIGGKQALETVQRLLPVLCQDHGL
TPDQVVAIASNNGGKQALETVQRLLPVLCQAHGLTPAQVVAIASNGGGKQAL
ETVQRLLPVLCQDHGLTPAQVVAIASHDGGKQALETVQRLLPVLCQAHGLTPE
QVVAIASHDGGKQALETVQRLLPVLCQAHGLTPEQVVAIASNGGGKQALETV
QRLLPVLCQAHGLTPAQVVAIASNGGGKQALETVQRLLPVLCQAHGLTPDQV
VAIASNNGGKQALETVQRLLPVLCQDHGLTPDQVVAIASNIGGKQALETVQRL
LPVLCQDHGLTPAQVVAIASNNGGKQALETVQRLLPVLCQDHGLTPAQVVAIA
SNIGGKQALETVQRLLPVLCQAHGLTPDQVVAIASNNGGKQALETVQRLLPVL
CQDHGLTPAQVVAIASNIGGKQALETVQRLLPVLCQAHGLTPEQVVAIASNNG
GKQALETVQRLLPVLCQAHGLTPEQVVAIASNGGGKQALESIVAQLSRPDPAL
AALTNDHLVALACLGGRPALDAVKKGLLVGSAIPVKRGATGETKVFTGNSNSP
KSPTKGGCSGSETPGTSESATPESSEVEFSHEYWMRHALTLAKRARDEREVPV
GAVLVLNNRVIGEGWNRAIGLHDPTAHAEIMALRQGGLVMQNYRLIDATLYVT
FEPCVMCAGAMIHSRIGRVVFGVRNSKRGAAGSLMNVLNYPGMNHRVEITEG
ILADECAALLCDFYRMPRQVFNAQKKAQSSIN
 (The underlined portion corresponds to the TALE protein including the N-terminal domain and C-terminal domain (including the half domain))
SEQ ID NO: 123. 
MASVLTPLLLRGLTGSARRLPVPRAKIHSLDYKDHDGDYKDHDIDYK
DDDDKGIRIQDLRTLGYSQQQEKIKPKVRSTVAQHHEALVGHGFTHAHIVAL
SQHPAALGTVAVKYQDMIAALPEATHEAIVGVGKYHGARALEALLTVAGELR
 
GPPLQLDTGQLLKIAKRGGVTAVEAVHAWRNALTGAPLNLTPAQVVAIASNIG
GKQALETVQRLLPVLCQAHGLTPEQVVAIASNNGGKQALETVQRLLPVLCQA
HGLTPDQVVAIASNGGGKQALETVQRLLPVLCQDHGLTPDQVVAIASHDGGK
QALETVQRLLPVLCQAHGLTPAQVVAIASHDGGKQALETVQRLLPVLCQDHG
LTPAQVVAIASNGGGKQALETVQRLLPVLCQDHGLTPAQVVAIASNGGGKQAL
ETVQRLLPVLCQAHGLTPAQVVAIASNNGGKQALETVQRLLPVLCQDHGLTPA
QVVAIASNIGGKQALETVQRLLPVLCQAHGLTPDQVVAIASNNGGKQALETVQ
RLLPVLCQDHGLTPAQVVAIASNIGGKQALETVQRLLPVLCQAHGLTPEQVVAI
ASNNGGKQALETVQRLLPVLCQAHGLTPAQVVAIASNIGGKQALETVQRLLPV
LCQAHGLTPAQVVAIASNNGGKQALETVQRLLPVLCQDHGLTPAQVVAIASNN
GGKQALETVQRLLPVLCQAHGLTPAQVVAIASNIGGKQALETVQRLLPVLCQA
HGLTPDQVVAIASNGGGKQALETVQRLLPVLCQAHGLTPDQVVAIASNGGGK
QALETVQRLLPVLCQAHGLTPEQVVAIASNGGGKQALESIVAQLSRPDPALAA
LTNDHLVALACLGGRPALDAVKKGLLVGSAIPVKRGATGETKVFTGNSNSPKSP
TKGGCSGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEP
CVMCAGAMIHSRIGRVVFGVRNSKRGAAGSLMNVLNYPGMNHRVEITEGILA
DECAALLCDFYRMPRQVFNAQKKAQSSIN
 (The underlined portion corresponds to the TALE protein including the N-terminal domain and C-terminal domain (including the half domain))
SEQ ID NO: 124. 
MASVLTPLLLRGLTGSARRLPVPRAKIHSLDYKDHDGDYKDHDIDYK
DDDDKGIRIQDLRTLGYSQQQEKIKPKVRSTVAQHHEALVGHGFTHAHIVAL
SQHPAALGTVAVKYQDMIAALPEATHEAIVGVGKYHGARALEALLTVAGELR
GPPLQLDTGQLLKIAKRGGVTAVEAVHAWRNALTGAPLNLTPDQVVAIASNGG
 
GKQALETVQRLLPVLCQAHGLTPAQVVAIASNGGGKQALETVQRLLPVLCQA
HGLTPEQVVAIASNGGGKQALETVQRLLPVLCQAHGLTPEQVVAIASNNGGKQ
ALETVQRLLPVLCQAHGLTPDQVVAIASNIGGKQALETVQRLLPVLCQDHGLT
PDQVVAIASNIGGKQALETVQRLLPVLCQAHGLTPAQVVAIASNNGGKQALET
VQRLLPVLCQDHGLTPAQVVAIASNGGGKQALETVQRLLPVLCQAHGLTPDQ
VVAIASHDGGKQALETVQRLLPVLCQAHGLTPAQVVAIASHDGGKQALETVQ
RLLPVLCQAHGLTPDQVVAIASNGGGKQALETVQRLLPVLCQAHGLTPAQVVA
IASNGGGKQALETVQRLLPVLCQDHGLTPDQVVAIASNNGGKQALETVQRLLP
VLCQDHGLTPAQVVAIASNIGGKQALETVQRLLPVLCQAHGLTPEQVVAIASN
NGGKQALETVQRLLPVLCQAHGLTPDQVVAIASNIGGKQALETVQRLLPVLCQ
DHGLTPAQVVAIASNNGGKQALETVQRLLPVLCQDHGLTPAQVVAIASNIGGK
QALETVQRLLPVLCQAHGLTPEQVVAIASNGGGKQALESIVAQLSRPDPALAA
LTNDHLVALACLGGRPALDAVKKGLLVGSAIPVKRGATGETKVFTGNSNSPKSP
TKGGCSGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEP
CVMCAGAMIHSRIGRVVFGVRNSKRGAAGSLMNVLNYPGMNHRVEITEGILA
DECAALLCDFYRMPRQVFNAQKKAQSSIN
 (The underlined portion corresponds to the TALE protein including the N-terminal domain and C-terminal domain (including the half domain))
SEQ ID NO: 125. 
MASVLTPLLLRGLTGSARRLPVPRAKIHSLDYKDHDGDYKDHDIDYK
DDDDKGIRIQDLRTLGYSQQQQEKIKPKVRSTVAQHHEALVGHGFTHAHIVAL
SQHPAALGTVAVKYQDMIAALPEATHEAIVGVGKYHGARALEALLTVAGELR
GPPLQLDTGQLLKIAKRGGVTAVEAVHAWRNALTGAPLNLTPAQVVAIASNNG
GKQALETVQRLLPVLCQDHGLTPAQVVAIASNGGGKQALETVQRLLPVLCQA
 
HGLTPDQVVAIASHDGGKQALETVQRLLPVLCQDHGLTPAQVVAIASHDGGK
QALETVQRLLPVLCQAHGLTPDQVVAIASNGGGKQALETVQRLLPVLCQAHG
LTPAQVVAIASNGGGKQALETVQRLLPVLCQDHGLTPDQVVAIASNNGGKQAL
ETVQRLLPVLCQDHGLTPAQVVAIASNIGGKQALETVQRLLPVLCQAHGLTPE
QVVAIASNNGGKQALETVQRLLPVLCQAHGLTPDQVVAIASNIGGKQALETVQ
RLLPVLCQDHGLTPAQVVAIASNNGGKQALETVQRLLPVLCQDHGLTPAQVVA
IASNIGGKQALETVQRLLPVLCQAHGLTPAQVVAIASNNGGKQALETVQRLLP
VLCQAHGLTPDQVVAIASNNGGKQALETVQRLLPVLCQDHGLTPAQVVAIASN
IGGKQALETVQRLLPVLCQAHGLTPEQVVAIASNGGGKQALETVQRLLPVLCQ
AHGLTPDQVVAIASNGGGKQALETVQRLLPVLCQDHGLTPDQVVAIASNIGGK
QALETVQRLLPVLCQDHGLTPEQVVAIASNGGGKQALESIVAQLSRPDPALAA
LTNDHLVALACLGGRPALDAVKKGLLVGSAIPVKRGATGETKVFTGNSNSPKSP
TKGGCSGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEP
CVMCAGAMIHSRIGRVVFGVRNSKRGAAGSLMNVLNYPGMNHRVEITEGILA
DECAALLCDFYRMPRQVFNAQKKAQSSIN
 (The underlined portion corresponds to the TALE protein including the N-terminal domain and C-terminal domain (including the half domain))

The combinations of the first fusion protein and the second fusion protein used in the base editors shown in FIG. 3 are as follows.

| Base Editor ID | First Fusion Protein | Second Fusion Protein |
|---|---|---|
| 306Nv + 120C | SEQ ID NO: 100 | SEQ ID NO: 118 |
| 310Nv + 385Cv | SEQ ID NO: 101 | SEQ ID NO: 119 |
| 310Nv + 120C | SEQ ID NO: 101 | SEQ ID NO: 118 |
| 314Nv + 120C | SEQ ID NO: 102 | SEQ ID NO: 118 |
| 318Nv + 397Cv | SEQ ID NO: 103 | SEQ ID NO: 120 |
| 318Nv + 385Cv | SEQ ID NO: 103 | SEQ ID NO: 119 |
| 318Nv + 120C | SEQ ID NO: 103 | SEQ ID NO: 118 |
| ZF6 97N + 120C | SEQ ID NO: 113 | SEQ ID NO: 118 |
| 322Nv + 385Cv | SEQ ID NO: 104 | SEQ ID NO: 119 |
| 322Nv + 120C | SEQ ID NO: 104 | SEQ ID NO: 118 |
| 322Nv + 115C | SEQ ID NO: 104 | SEQ ID NO: 121 |
| 322Nv + 110C | SEQ ID NO: 104 | SEQ ID NO: 122 |
| 90N + 397Cv | SEQ ID NO: 105 | SEQ ID NO: 120 |
| 90N + 389Cv | SEQ ID NO: 105 | SEQ ID NO: 123 |
| 90N + 385Cv | SEQ ID NO: 105 | SEQ ID NO: 119 |
| 90N + 120C | SEQ ID NO: 105 | SEQ ID NO: 118 |
| 90N + 115C | SEQ ID NO: 105 | SEQ ID NO: 121 |
| 90N + 110C | SEQ ID NO: 105 | SEQ ID NO: 122 |
| 326Nv + 385Cv | SEQ ID NO: 106 | SEQ ID NO: 119 |
| 326Nv + 120C | SEQ ID NO: 106 | SEQ ID NO: 118 |
| 326Nv + 115C | SEQ ID NO: 106 | SEQ ID NO: 121 |
| 326Nv + 110C | SEQ ID NO: 106 | SEQ ID NO: 122 |
| 326Nv + 381Cv | SEQ ID NO: 106 | SEQ ID NO: 124 |
| ZF1 97N + 397Cv | SEQ ID NO: 111 | SEQ ID NO: 120 |
| ZF1 97N + 120C | SEQ ID NO: 111 | SEQ ID NO: 118 |
| 330Nv + 120C | SEQ ID NO: 107 | SEQ ID NO: 118 |
| 334Nv + 397Cv | SEQ ID NO: 108 | SEQ ID NO: 120 |
| 334Nv + 385Cv | SEQ ID NO: 108 | SEQ ID NO: 119 |
| 334Nv + 120C | SEQ ID NO: 108 | SEQ ID NO: 118 |
| 334Nv + 115C | SEQ ID NO: 108 | SEQ ID NO: 121 |
| 334Nv + 110C | SEQ ID NO: 108 | SEQ ID NO: 122 |
| 334Nv + 381Cv | SEQ ID NO: 108 | SEQ ID NO: 124 |
| ZF5 97N + 397Cv | SEQ ID NO: 112 | SEQ ID NO: 120 |
| ZF5 97N + 120C | SEQ ID NO: 112 | SEQ ID NO: 118 |
| ZF5 97N + 115C | SEQ ID NO: 112 | SEQ ID NO: 121 |
| ZF5 97N + 381Cv | SEQ ID NO: 112 | SEQ ID NO: 124 |
| ZF6 97C + 389Nv | SEQ ID NO: 114 | SEQ ID NO: 115 |
| ZF6 97C + 385Nv | SEQ ID NO: 114 | SEQ ID NO: 116 |
| 322Cv + 393Nv | SEQ ID NO: 109 | SEQ ID NO: 117 |
| 322Cv + 389Nv | SEQ ID NO: 109 | SEQ ID NO: 115 |
| 322Cv + 385Nv | SEQ ID NO: 109 | SEQ ID NO: 116 |
| 90C + 389Nv | SEQ ID NO: 110 | SEQ ID NO: 115 |

## Claims

1. A base editing composition capable of correcting a mitochondrial DNA mutation in a patient with Leber hereditary optic neuropathy (LHON), comprising one or more fusion proteins, wherein each of the one or more fusion proteins independently comprises DNA binding protein that specifically binds to mitochondrial DNA of a patient with LHON and further comprises at least one of adenine deaminase and cytosine deaminase, and wherein cytosine deaminase is present in a full-length form or in the form of two splits.

2. The base editing composition according to claim 1, wherein the composition is capable of editing adenine (A) at position 3460 of mitochondrial ND1 DNA to guanine (G), adenine (A) at position 11778 of mitochondrial ND4 DNA to guanine (G), or cytosine (A) at position 14484 of mitochondrial ND6 DNA to thymine (T) in a patient with LHON.

3. The base editing composition according to claim 1 or 2, wherein cytosine deaminase is APOBEC (apolipoprotein B editing complex), AID (activation-induced deaminase), TadA (tRNA-specific adenosine deaminase), or DddAtox, or a variant thereof.

4. The base editing composition according to claim 1 or 2, wherein cytosine deaminase is DddAtox and is included in the form of a first split and a second split, and wherein one or more amino acids located on the interface between the first and second splits are substituted with other amino acids.

5. The base editing composition according to claim 4, wherein the first split of DddAtox comprises an amino acid sequence of SEQ ID NO: 5 or a variant thereof, said variant comprising substitution of one or more amino acids selected from the group consisting of positions 87, 88, 91, 92, 95, 100, 101, 102, and 103 of SEQ ID NO: 5 with other amino acids, and wherein the second split of DddAtox comprises an amino acid sequence of SEQ ID NO: 6 or a variant thereof, said variant comprising substitution of one or more amino acids selected from the group consisting of positions 13, 14, 15, and 16 of SEQ ID NO: 6 with other amino acids.

6. The base editing composition according to claim 1 or 2, wherein adenine deaminase is APOBEC, AID, or TadA, or a variant thereof.

7. The base editing composition according to claim 6, wherein adenine deaminase comprises an amino acid sequence of SEQ ID NO: 1 or a conservative amino acid substitution thereof.

8. The base editing composition according to any one of claims 1 to 7, wherein DNA binding protein is selected from the group consisting of zinc finger protein, TALE protein, and CRISPR-associated nuclease.

9. The base editing composition according to any one of claims 1 to 8, wherein each of the one or more fusion proteins independently comprises uracil glycosylase inhibitor (UGI).

10. The base editing composition according to any one of claims 1 to 9, wherein each of the one or more fusion proteins independently comprises nuclear export signal (NES).

11. The base editing composition according to any one of claims 1 to 10, wherein each of the one or more fusion proteins independently comprises mitochondrial targeting sequence (MTS).

12. The base editing composition according to any one of claims 1 to 8, wherein one DNA binding protein binds to a nucleotide sequence of 5'-CAAACTCAAACTACGAACGCACTCACAGTCACATCATAATCCTCTCTCAAGGACTT CAAAC-3' or a portion thereof of mitochondrial ND4 DNA.

13. The base editing composition according to any one of claims 1 to 8, wherein one DNA binding protein binds to a nucleotide sequence of 5'-CAAACTCAAACTACGAACGCACTCACAGTCACATCATAATCCTCTCTCAAGGACTT CAAAC-3' or a portion thereof of mitochondrial ND4 DNA.

14. The base editing composition according to any one of claims 1 to 8, wherein one DNA binding protein binds to a nucleotide sequence of 5'-TCGCTGTAGTATATCCAAAGACAACCACCATTCCCCCTAAATAAATTAAAAAAACT-3' or a portion thereof mitochondrial ND6 DNA.

15. The base editing composition according to any one of claims 1 to 8, wherein one DNA binding protein comprises an amino acid sequence selected from the group consisting of SEQ ID NOs: 7 to 65, or a conservative amino acid substitution thereof.

16. The base editing composition according to claim 1, wherein the composition comprises two fusion proteins and is capable of editing adenine (A) at position 3460 of mitochondrial ND1 DNA to guanine (G) in a patient with LHON, wherein each of the two fusion proteins comprises DddAtox split and TALE protein that specifically binds to mitochondrial ND1 DNA, and one of the two fusion proteins further comprises TadA8e.

17. The base editing composition according to claim 1, wherein the composition comprises two fusion proteins and is capable of editing adenine (A) at position 11778 of mitochondrial ND4 DNA to guanine (G) in a patient with LHON, wherein each of the two fusion proteins comprises DddAtox split and TALE protein or zinc finger protein that specifically binds to mitochondrial ND4 DNA, and one of the two fusion proteins further comprises TadA8e.

18. The base editing composition according to claim 1, wherein the composition comprises two fusion proteins and is capable of editing cytosine (A) at position 14484 of mitochondrial ND6 DNA to thymine (T) in a patient with LHON, wherein each of the two fusion proteins comprises DddAtox split and TALE protein that specifically binds to mitochondrial ND6.

19. The base editing composition according to claim 16, wherein one fusion protein comprises TALE protein comprising an amino acid sequence selected from the group consisting of SEQ ID NOs: 10 to 17 or conservative amino acid substitution thereof, and the other fusion protein comprises TALE protein comprising an amino acid sequence selected from the group consisting of SEQ ID NOs: 18 to 31 or a conservative amino acid substitution thereof.

20. The base editing composition according to claim 17, wherein one fusion protein comprises TALE protein comprising an amino acid sequence selected from the group consisting of SEQ ID NOs: 32 to 42 or zinc finger protein comprising an amino acid sequence selected from the group consisting of SEQ ID NOs: 7 to 9 or conservative amino acid substitution thereof, and the other fusion protein comprises TALE protein comprising an amino acid sequence selected from the group consisting of SEQ ID NOs: 43 to 53 or zinc finger protein comprising an amino acid sequence selected from the group consisting of SEQ ID NOs: 7 to 9 or a conservative amino acid substitution thereof.

21. The base editing composition according to claim 18, wherein one fusion protein comprises TALE protein comprising an amino acid sequence selected from the group consisting of SEQ ID NOs: 54 to 57 or conservative amino acid substitution thereof, and the other fusion protein comprises TALE protein comprising an amino acid sequence selected from the group consisting of SEQ ID NOs: 58 to 65 or a conservative amino acid substitution thereof.

22. The base editing composition according to claim 16 or 19, wherein the composition comprises a combination of a fusion protein comprising an amino acid sequence selected from the group consisting of SEQ ID NOs: 78 to 85 and a fusion protein comprising an amino acid sequence selected from the group consisting of SEQ ID NOs: 86 to 99.

23. The base editing composition according to claim 17 or 20, wherein the composition comprises a combination of a fusion protein comprising an amino acid sequence selected from the group consisting of SEQ ID NOs: 100 to 114 and a fusion protein comprising an amino acid sequence selected from the group consisting of SEQ ID NOs: 115 to 125.

24. The base editing composition according to claim 18 or 21, wherein the composition comprises a combination of a fusion protein comprising an amino acid sequence selected from the group consisting of SEQ ID NOs: 66 to 69 and a fusion protein comprising an amino acid sequence selected from the group consisting of SEQ ID NOs: 70 to 77.

25. A polynucleotide encoding any one of the fusion proteins included in the base editing composition according to any one of claims 1 to 24, or a combination of two or more of said polynucleotides.

26. The combination of polynucleotides according to claim 25, wherein the combination of polynucleotides comprises a polynucleotide encoding an amino acid sequence selected from the group consisting of SEQ ID NOs: 78 to 85 and a polynucleotide encoding an amino acid sequence selected from the group consisting of SEQ ID NOs: 86 to 99.

27. The combination of polynucleotides according to claim 25, wherein the combination of polynucleotides comprises a polynucleotide encoding an amino acid sequence selected from the group consisting of SEQ ID NOs: 100 to 114 and a polynucleotide encoding an amino acid sequence selected from the group consisting of SEQ ID NOs: 115 to 125.

28. The combination of polynucleotides according to claim 25, wherein the combination of polynucleotides comprises a polynucleotide encoding an amino acid sequence selected from the group consisting of SEQ ID NOs: 66 to 69 and a polynucleotide encoding an amino acid sequence selected from the group consisting of SEQ ID NOs: 70 to 77.

29. A method of correcting a mitochondrial DNA mutation in a patient with LHON, the method comprising contacting mitochondrial DNA of a patient with LHON with a base editing composition according to any one of claims 1 to 24, wherein the correction involves editing of guanine (G) at position 3460 of mitochondrial ND1 DNA to adenine (A), guanine (G) at position 11778 of mitochondrial ND4 DNA to adenine (A), or cytosine (A) at position 14484 of mitochondrial ND6 DNA to thymine (T).

30. A method of preventing or treating LHON, comprising administering to a patient in need of prevention or treatment of LHON a base editing composition according to any one of claims 1 to 24 or a composition comprising a polynucleotide or a combination of polynucleotides according to any one of claims 25 to 28.

31. A pharmaceutical composition for preventing or treating LHON, comprising a base editing composition according to any one of claims 1 to 24 or a polynucleotide or a combination of polynucleotides according to any one of claims 25 to 28.

32. A gene delivery vehicle comprising a polynucleotide or a combination of polynucleotides according to any one of claims 25 to 28.

33. The gene delivery vehicle according to claim 32, wherein the gene delivery vehicle is an adeno-associated virus vector.

34. The gene delivery vehicle according to claim 32, wherein the gene delivery vehicle is a lipid nanoparticle or a polymeric nanoparticle.

35. A gene therapy agent for preventing or treating LHON, comprising a gene delivery vehicle according to claim 32.
